Europäisches Patentamt

European Patent Office

Office européen des brevets

(19)

(11) Veröffentlichungsnummer: **0 319 058**
**A1**

## (12) EUROPÄISCHE PATENTANMELDUNG

(21) Anmeldenummer: 88202476.3

(22) Anmeldetag: 04.11.88

(51) Int. Cl.⁴: **A61K 7/06**

(30) Priorität: 03.12.87 NL 8702913

(43) Veröffentlichungstag der Anmeldung:
07.06.89 Patentblatt 89/23

(84) Benannte Vertragsstaaten:
AT BE CH DE ES FR GB GR IT LI LU NL SE

(71) Anmelder: VIALLE MINERALS B.V.
Hakkesstraat 39
NL-5916 PX Venlo(NL)

(72) Erfinder: Vialle, Joseph Engelbert Christiaan
Grijzendijk 12
NL-5916 PP Venlo(NL)

(74) Vertreter: Baarslag, Aldert D. et al
Nederlandsch Octrooibureau
Scheveningseweg 82 P.O. Box 29720
NL-2502 LS 's-Gravenhage(NL)

(54) Haarwuchsmittel.

(57) Die Erfindung betrifft ein Mittel zur Förderung des Haarwuchses, das pflanzenartige Extrakte enthält. Die pflanzenartigen Extrakte stammen aus Sichuan Pericarpium zanthoxyli, Radix Angelicae sinensis, Cacumen biotae, Folium mori, Rhizoma Zingiberis recens, Radix Stemonae, Fructus Grataegi, Flos Carthami, Fructus Capsici, Folium Sesami und Cortex Pseudolaricis. Weiter sind in der Lösung in Äthanol und Wasser Quecksilbersulfat, Bleisulfat und Arsensulfat enthalten. Das Mittel wird auf der Haupthaut appliziert.

EP 0 319 058 A1

## Haarwuchsmittel

Die Erfindung betrifft ein Mittel zur Förderung des Haarwuchses, das pflanzenartige Extrakte enthält.

Mittel zur Haarwuchsförderung, die pflanzenartige Extrakte enthalten sind aus einer grossen Anzahl von Veröffentlichungen bekannt.

Aus der luxemburgischen Patentanmeldung 67 493 ist ein Mittel zur Haarwuchsförderung bekannt, dass aus einer kapilläraktiven Lotion, die Urtica Urens oder Urtica Dioica Tinktur, Capsicum Tinktur, Senfkeimentinktur und weiter Hyaluronidase und ein verdickungsmittel enthält.

Aus der französischen Patentschrift 2 539 621 ist ein Haarwuchsmittel bekannt, dass aus drei Lotions besteht, wobei die erste Lotion Ginseng Tinktur und Astragalus Tinktur, die zweite Lotion u.a. Zingiber Officinalis und Sesamöl und die dritte Lotion Astragalus und Angélique Polymorpha enthält. Während der Behandlung werden diese drei Lotions nacheinander angewandt.

Aus der französischen Patentanmeldung 2 558 727 ist es bekannt Haar oder die Haupthaut mit Cyprèsextrakt, Thuyaextrakt, Juniperusextrakt, Lobocedrus, Extrakt der Cupressaceën, Essig und Wasser zu behandeln.

Aus der deutschen Offenlegungsschrift 2 649 846 ist es bekannt ein Haarbehandlungsmittel anzuwenden, das Paprika in einem Lösungsmittel enthält. Als Lösungsmittel wird hierbei vorzugsweise Spiritus oder Alkohol angewandt.

Es ist jetzt gefunden worden, dass ein Mittel zur Förderung des Haarwuchses, das pflanzenartige Extrakte aufweist, dadurch gekennzeichnet wird, dass dieses Mittel hergestellt ist auf Basis eines Extraktes erhältlich durch Extraktion von:

3 - 7 Gew. Tln Sichuan Pericarpium zanthoxyli;

7 - 11 Gew. Tln Radix Angelicae sinensis;

7 - 11 Gew. Tln Cacumen biotae;

4 - 8 Gew. Tln Folium mori;

7 - 11 Gew. Tln Rhizoma Zingiberis recens;

0,5 - 1,5 Gew. Tln Radix Stemonae

15 - 25 Gew. Tln Fructus Crataegi;

2,5 - 3,5 Gew. Tln Flos.Carthami;

0,75 - 1,25 Gew.Tln Fructus Capsici;

4 - 8 Gew. Tln Folium Sesami;

2,5 - 3,5 Gew. Tln Cortex Pseudolaricis;

mit 100 Gew. Tln eines Gemisches aus 50-70 Gew. % Äthanol und 30-50 Gew.% Wasser und worin

1,5 - 2,5 mg Quecksilbersulfat;

7,5 - 12,5 mg Bleisulfat und

2,5 - 3,5 mg Arsensulfat pro 100 ml und gegebenenfalls weitere Verdünnungsmittel, Riechstoffe, Farbstoffe und/oder Verdickungsmittel aufgenommen worden sind.

Vorzugsweise wird ein Extrakt angewandt, erhalten durch die nachfolgende Zusammensetzung:

4,5 - 5,5 Gew. Tln Sichuan Pericarpium zanthoxyli;

8,5 - 9,5 Gew. Tln Radix Angelicae sinensis;

8,5 - 9,5 Gew. Tln Cacumen biotae;

5,5 - 6,5 Gew. Tln Folium Mori;

8,5 - 9,5 Gew. Tln Rhizoma Zingiberis recens;

0,9 - 1,1 Gew. Tln Radix Stemonae;

19 - 21 Gew. Tln Fructus Crataegi;

2,8 - 3,2 Gew. Tln Flos. Carthami;

0,9 - 1,1 Gew. Tln Fructus Capsici;

5,5 - 6,5 Gew. Tln Folium Sesami;

2,9 - 3,1 Gew. Tln Cortex Pseudolaricis;

mit 100 Volumenteilen eines Äthanol-Wasser-Gemisches zu extrahieren und daran:

1,9 - 2,1 mg Quecksilbersulfat;

9,5 - 10,5 mg Bleisulfat

2,9 - 3,1 mg Arsensulfat zuzusetzen.

Sehr insbesonder wird ein Extrakt aus:

5 Gew. Tln Sichuan Pericarpium zanthoxyli;

9 Gew. Tln Radix Angelicae sinensis;

9 Gew. Tln Cacumen biotae;

6 Gew. Tln Folium mori;

9 Gew. Tln -Rhizoma Zingiberis recens;

1 Gew. Tl Radix Stemonae;

20 Gew. Tln Fructus Crataegi;

3 Gew. Tln Flos. Carthami;

1 Gew. Tl Fructus Capsici;

6 Gew. Tln Folium Sesami;

3 Gew. Tln Cortex Pseudolaricis;

mit 100 Volumenteilen eines Äthanol-Wassergemisches woran:

0,02 mg Quecksilbersulfat;

0,1 mg Bleisulfat;

0,03 mg Arsensulfat zugesetzt worden sind, angewandt.

Diese Zusammensetzung wird in 60-70 Gew. % Äthanol und 30-40 Gew. % Wasser aufgenommen.

Das Extrakt wird hergestellt durch die angegebene Menge an Kräuter zu extrahieren in 100 ml wasserenthaltendes Äthanol mit einem Äthanolgehalt von 60-70 Gew.%. Die Kräuter werden hier während einer Woche auf 0-35° C gehalten. Obgleich selbstverständlich eine kürzere und längere Zeit auch möglich ist.

Dem erfindungsgemässen Mittel können die üblichen Hilfstoffen zugesetzt werden. Weiter kann eine kleine Menge an Verdickungsmittel aufgenommen werden.

Das erfindungsgemässe Mittel wird durch Einreiben auf die Haut in einer Menge von etwa 3,5 ml jedes Mal angewandt, und wohl zwei Mal am Tag. Diese Anwendung verursacht eine erhebliche Anregung des Haarwuchses.

Das erfindungsgemässe Mittel kann in einfacher Weise hergestellt werden durch die Salze aufzulösen in einem Kräuterextrakt in Äthanol und Wasser. Zweckmässig ist hier ein Gemisch aus 60-70 Gew.% Äthanol und 30-40 Gew.% Wasser.

Beispiel I

Man extrahiert 5 Gew. Tln Sichuan Pericarpium zanthoxyli;

9 Gew. Tln Radix Angelicae sinensis (chinesische Angelica);

9 Gew. Tln Cacumen Biotae (Zweige mit Laub der chinesischen Thuja);

6 Gew. Tln Folium Mor (Malbeerblatt);

9 Gew. Tln Bhizoma Zingiberis Recens (frische Ingwer);

1 Gew. Tl Radix Stemonae (Wurzel der ungestielten Stemonae);

20 Gew. Tln Fructus Crataegi (Hagedornfrüchte);

3 Gew. Tln Flos. Carthami (Safranblume);

1 Gew. Tl Fructus Capsici (heisse Pfeffer);

6 Gew. Tln Folium Sesami (Sesamblatt);

3 Gew. Tln Cortex Pseudolaricis (pseudo Lariks);

während einer Woche bei Zimmertemperatur in einem Gemisch aus 65 Gew. %Äthanol und 35 Gew.% Wasser und setzt daran zu:

0,02 mg Quecksilbersulfat;

0,1 mg Bleisulfat;

0,03 mg Arsensulfat.

In einfacher Weise können Gemische mit einer etwas anderer Zusammensetzung innerhalb der angegebenen Trajekte hergestellt werden.

Überprüfung der Sicherheit des Haarwuchsmittels.

1. Die Sicherheit des Mittels ist überprüft worden durch:
   a) Versuche nach akuter Toxizität;
   b) Versuche bezüglich chronischer Toxizität;
   c) Versuche nach der Pathologie;
   d) Versuche nach perkutaner Toxizität;
   e) Versuche nach Kontaktsensibilisierung;

f) Versuche nach Sleimhautirritation:

g) Koagulationsversuche;

h) Hämolyseversuche;

i) Mikrobiologische Versuche (Bakterien, Fungi, Hefepilze)

## 2. Versuchsmaterial und Versuchsdauer.

a) Versuchsmaterial: das Mittel hergestellt gemäss Beispiel I.

b) Verpackung: 100 ml braune visköse Flüssigkeit in schwarzen Polybins(Behältern) mit einem Inhalt von 150 ml, bei Anwendung wird pro Mal 1-2 ml des Versuchsmaterials aus einer Öffnung von dem Typ Spritzmund abgegeben.

## 3. Erfolge.

a) Versuche nach akuter Toxizität.

Eine Gruppe von 20 männlichen Mäusern des DD Stammes mit einem Körpergewicht von 16-17 g ist angewandt worden und eine experimentelle Lösung und eine blanko Lösung (Lösung von Traubenzucker) wurden in einer Menge von 5, 50, 100, 500 und 1000 ml in die Bauchhöhle und in die Haargefässe eingebracht und die Anzahl der Toten ist in der vierten Woche nach dem Einspritzen bestimmt worden.

**Tabelle A. Versuche nach der akuten Toxizität nach dem Einspritzen in die Bauchhöhle von Mäusern.**

| Eingespritzte Menge | Beurteilungsperiode (Tage) | | | | | | | | |
|---|---|---|---|---|---|---|---|---|---|
| | 1 | 2 | 3 | 4 | 5 | 6 | 14 | 21 | 28 |
| 5 ml | 0/20* | 0/20 | 0/20 | 0/20 | 0/20 | 0/20 | 0/20 | 0/20 | 0/20 |
| 50 ml | 0/20 | 0/20 | 0/20 | 0/20 | 0/20 | 0/20 | 0/20 | 0/20 | 0/20 |
| 100 ml | 0/20 | 0/20 | 0/20 | 0/20 | 0/20 | 0/20 | 0/20 | 0/20 | 0/20 |
| 500 ml | 0/20 | 0/20 | 0/20 | 0/20 | 0/20 | 0/20 | 0/20 | 0/20 | 0/20 |
| 1000 ml | 0/20 | 0/20 | 0/20 | 0/20 | 0/20 | 0/20 | 0/20 | 0/20 | 0/20 |
| blanko | | | | | | | | | |

\* Anzahl der Toten / Anzahl der Versuche

Tabelle B.

| Versuche nach akuter Toxizität nach Einspritzen in die Haargefässe von Mäusern. | | | | | | | | |
|---|---|---|---|---|---|---|---|---|
| Eingespritzte Menge | Beurteilungsperiode (Tage) | | | | | | | |
| | 1 | 2 | 3 | 4 | 5 | 6 | 14 | 2 |
| 5 ml | 0/20* | 0/20 | 0/20 | 0/20 | 0/20 | 0/20 | 0/20 | 0/20 |
| 50 ml | 0/20 | 0/20 | 0/20 | 0/20 | 0/20 | 0/20 | 0/20 | 0/20 |
| 100 ml | 0/20 | 0/20 | 0/20 | 0/20 | 0/20 | 0/20 | 0/20 | 0/20 |
| 500 ml | 0/20 | 0/20 | 0/20 | 0/20 | 0/20 | 0/20 | 0/20 | 0/20 |
| 1000 ml | 0/20 | 0/20 | 0/20 | 0/20 | 0/20 | 0/20 | 0/20 | 0/20 |

\* Anzahl der Toten / Anzahl der Versuche

Aus dem obigen Erfolg wurde keine akute Toxizität nach Einspritzen in die Bauchhöhle und in die Haargefässe in einer Menge bis 1000 ml wahrgenommen.

b) Versuche nach chronischer Toxizität.

Versuche nach chronischer Toxizität sind durchgeführt worden mit festem Futter das 100 ml, 500 ml, 1000 ml und 2000 ml/g der Versuchsflüssigkeit und blanko Flüssigkeit (destilliertes Wasser als blanko Flüssigkeit) enthielt mit als Gruppe 20 männlichen Mäusern des DD Stammes mit einem Körpergewicht von 16-17 g und die Beurteilung ist durchgeführt worden auf Basis der Anzahl der Toten.

**Tabelle C. Versuche nach chronischer Toxizität des Haarwuchsmittels.**

| Eingespritzte | Beurteilungsdauer (Tage) | | | | | |
|---|---|---|---|---|---|---|
| Menge | 1 | 2 | 3 | 4 | 5 | 6 |
| 100 ml | 0/20* | 0/20 | 0/20 | 0/20 | 0/20 | 0/20 |
| 500 ml | 0/20 | 0/20 | 0/20 | 0/20 | 0/20 | 0/20 |
| 1000 ml | 0/20 | 0/20 | 0/20 | 0/20 | 0/20 | 0/20 |
| 2000 ml | 0/20 | 0/20 | 0/20 | 0/20 | 0/20 | 0/20 |
| blanko | 0/20 | 0/20 | 0/20 | 0/20 | 0/20 | 0/20 |

**\* Anzahl der Toten / Anzahl der Versuche**

c) Pathologische Versuche

Bei den Versuchsmäusern, die in den Versuchen nach chronischer Toxizität angewandt wurden und bei den blanko Mäusern wurde immer drei Mäuser pro Gruppe genommen, und von den toten Mäusern werden üblicher Weise nach Fixieren mit Formalin Parrafincoups hergestellt und die Bilder der Gewebeänderung von mit Hämatoxolin-eosin-gefärbten Spezimen wurden beurteilt.

Erfolg:

Entzündungsreaktionen von mehreren Geweben und Abnormalitäten von neurophilen Lymphozyten und Histiozyten sind nicht wahrgenommen worden und Gewebeschwellung und es gibt das gleiche Gewebebild als beim blanko Versuch gefunden worden ist.

d) Versuche nach perkutaner Toxizität.

Dem Vaselin wurde soviel Mittel gemäss Beispiel auch zugesetzt, dass die Konzentration davon 1 Gew.%, 2 Gew.%, 5 Gew.%, 10 Gew.%, 20 Gew.% und 50 Gew.% wurde und die in dieser Weise präparierte Flüssigkeit wurde homogen auf ein Karree von 1 cm2 auf die erst rasierte Haut von Mehrschweinchen mit einem Körpergewicht von 250 g appliziert. Nach 30 Minuten wurde nach der Lösung der Epidermis, rote Fleckigkeit und Schwellung observiert.

Weiter wurden alle Versuche durchgeführt mit Mehrschweinchen und wurde als blanko Versuch nur Vaselin observiert.

Erfolg:

Wie in Tabelle D gezeigt wird, war die Wahrnehmung bei einer Konzentration von 1 bis 50% für die Lösung der Epidermis, rote Fleckigkeit und Schwellung völlig negativ.

Tabelle D.

| Versuche nach perkutaner Toxizität des Haarwuchsmittels. | | | | | | |
|---|---|---|---|---|---|---|
| Konzentration (%) Zeit der Observation der perkutaner Toxizität | | | | | | |
| | 24 Stunden | | | 48 Stunden | | |
| | Epidermis | rote Flecke | Schwellung | Epidermis | rote Flecke | Schwellung |
| 1 | - | - | - | - | - | - |
| 2 | - | - | - | - | - | - |
| 5 | - | - | - | - | - | - |
| 10 | - | - | - | - | - | - |
| 20 | - | - | - | - | - | - |
| 50 | - | - | - | - | - | - |
| blanko (Vaselin) | - | - | - | - | - | - |
| -: negativ | | | | | | |

e) Versuche nach Kontaktsensibilisierung.

1 ml der ursprünglichen Flüssigkeit wurde 3 Mal am Tag und das während 5 Tage wiederhohlt auf ein Teil der erst rasierten Haut am Rücken von 2 männlichen Mehrschweinchen mit einem Körpergewicht von 200 g aufgebracht, und die klinische Phänomene wurden studiert.

Erfolg:

Der Erfolg der Durchführung der Kontaktsensibilisierung die 3 Mal am Tag wiederhohlt wurde war dass bei der Wahrnehmung während 5 Tage keine Schwellungssymptome und rote Flecke der Haut wahrgenommen wurden, und dass auch aufgrund von klinischen pathologischen Befindungen keine Abnormalitäten wahrgenommen wurden.

f) Versuche nach Schleimhautirritation.

Diese wurden mittels Augenreaktionen durchgeführt. Drei männliche österreichische Kaninchen von 2,3 kg die erst während 5 Tage auf 25° C gehandhabt wurden, wurden angewandt. In 1 Auge wurde 1 Tropf Dabao getropft und nach 20 Minuten wurden An- oder Abwesenheit von Blutkongestionen, Strömung von Tränen und Ödem untersucht.

Erfolg:

Bei den drei Versuchskaninchen und 1 blanko, die mit destilliertem Wasser behandelt wurden, wurden 25 Minuten nach dem Tropfen keine Blutkongestionen und Ödem im Augeteil wahrgenommen.

g) Koagulationsversuche

Trockenes Plasma von Kaninchen und mensliches Plasma der Blutgruppe 0 wurden hergestellt und mit 0,5 ml Versuchsflüssigkeit wurde je 1 ml Plasma vermischt, wonach man dies 2 Stunden bei 37° C reagieren lässt, und das koagulierendes Vermögen davon observiert wurde.

Erfolg:

In diesen Versuchen wurde beim Plasma von Kaninchen und bei menschlichem Plasma ein Koagulationsvermögen wahrgenommen.

h) Hämolyseversuch.

Frische Blutkörperchen von Kaninchen und Menschen wurden angewandt und das Versuchsmaterial wurde in Konzentrationen von 0,1, 0,2, 0,5 und 1,0 Gew.% mit 0,5 ml Blutkörperchen von Kaninchen und Menschen vermischt und man lässt dies bei 37° C reagieren.

Tabelle E.

| Konzentration des Versuchsmaterials. | | |
|---|---|---|
| (%) | Kaninchen | Mensche |
| 0,1 | -* | - |
| 0,2 | - | - |
| 0,5 | - | - |
| 1,0 | - | - |

* : keine Hämolyse.

i) Mikrobiologische Versuche.

Als Nahrboden für die Detektion von Bakterien wurde Trypto-soja Agar angewandt, für Fungi Kartoffeldextrose und als Nahrboden für die Detektion von Schimmelpilzen einen Saburow Agar Nahrboden, und die Kultur wurde bei Kulturtemperaturen von 30° und 37° C durchgeführt. Weiter wurde die Untersuchung durchgeführt mit als Nahrboden für die Detektion von Darmbakterien einen Nahrboden von MacConki, und einem Mannit und Trypto-Soja-Blutplasma Agar Nahrboden. Die Kultur wurde durchgeführt, während aseptisch 1 ml aller Materialien auf die obengenannten Nahrböden ausgeschmiert wurden.

Tabelle F.

| Mikrobiologische Versuche mit dem Haarwuchsmittel auf mehrere Nahrböden. | | | | | | |
|---|---|---|---|---|---|---|
| Nahrboden | Los Nr. | | | | | |
| | 1-8-86 | | 2-8-86 | | 3-8-86 | |
| | 30°C | 37°C | 30°C | 37°C | 30°C | 37°C |
| Tryptokase Soja | - a) | - | - | - | - | - |
| MacConki | - | - | - | - | - | - |
| Mannit | - | - | - | - | - | - |
| Kartoffeldextrose | | - | - | - | - | - |
| Saburow | | - | - | - | - | |

a): Detektion von mikroorganismen negativ.

Erfolg:

Wie aus der obigen Tabelle hervorgeht wurden keine Mikroorganismen wahrgenommen auf die unterschiedlichen Nahrböden bei 30°C und 37°C.

Eine Untersuchung nach dem Haarwuchsmechanismus des Haarwuchsmittels mittels des "laser Doppler" Blutstrommessers (PF2).

Bei einer grossen Anzahl von Haarausfall-Leitenden im In- und Ausland kann der Anwendung des Haarwuchsmittels dafür sorgen, dass an den Stellen wo Haarausfall aufgetreten ist in schnellerem Tempo neue Haare wachsen.

Für die weitere Observation der therapeutischen Effekte dieses Mittels ist gleicherzeit eine Untersuchung durchgeführt worden nach dem Einfluss die es auf den Haarwuchsmechanismus hat.

Mittels des neuen Models "laser-Doppler"-Blutstrommesser, das in den Jahren 80 entwickelt worden ist, sind bei 301 Fällen von Haarausfall-Leidenden, die eine systematische Behandlung mit dem Haarwuchsmittel untergingen Wahrnehmungen auf mehrere Punkte durchgefürht worden nach der Durchstromkapazität der kapillären Gefässe auf die Haupthaut um die Zusammenhang zwischen Haarwuchs und Blutdurchstromkapazität der kapillären Gefässe der Haupthaut zu untersuchen.

Allgemeine Angaben.

Poliklinisch sind 301 Fälle von Leidenden an Haarausfall selektiert worden, wovon 258 vom männlichen Geschlecht und 43 vom weiblichen Geschlecht, im Alter variierend von 17 bis 52 Jahren (Tabelle G). Die kürzeste Zeit der Erkrankung war 2 Monate, die längste war 23 Jahre (Tabelle H). Es waren 24 Fälle völlig kahl, 10 Fälle waren teilweise kahl und bei 267 Fällen war die Kahlheit verursacht durch Seborrhoea; letztgenannte Fälle werden für die Behandlung entsprechend der Hamilton Methode in Typen aufgeteilt (Tabelle I).

Tabelle G.

| Verteilung von 301 Patienten nach Alter. | | | | | | | | |
|---|---|---|---|---|---|---|---|---|
| | Geschlecht | | Alter (Jahre) | | | | | |
| | Mann | Frau | ≤20 | 21~30 | 31~40 | 41~50 | 51~60 | Gesamt |
| Anzahl | 258 | 43 | 5 | 62 | 122 | 106 | 6 | 301 |
| Prozente | 85.7 | 14.3 | 1.7 | 20.6 | 40.5 | 35.2 | 2.0 | 100 |

Tabelle H.

| Zeitsdauer der Erkrankung bei den 301 Patienten. | | | | | | | |
|---|---|---|---|---|---|---|---|
| | <1/2 J | <3J | <5J | <10J | <15J | <20J | >20J |
| Anzahl | 9 | 26 | 28 | 86 | 90 | 34 | 28 |
| Prozente | 2.99 | 18.64 | 9.30 | 28.57 | 29.90 | 11.30 | 9.30 |

Tabelle I.

| Die verschiedenen Kategorien von Haarausfall bei den 301 Patienten. | | | | | | | | | | |
|---|---|---|---|---|---|---|---|---|---|---|
| | Völlige Kahlheit | Lokale Kahlheit | Haarausfall wegen Soborrhoea | | | | | | | Gesamt |
| | | | H2 | H3 | H4 | H5 | H6 | H7 | H8 | |
| Anzahl | 24 | 10 | 13 | 39 | 56 | 41 | 41 | 40 | 37 | 301 |
| Prozente | 8.0 | 3.3 | 4.3 | 13.0 | 18.6 | 13.6 | 16.6 | 13.3 | 12.3 | 100 |

## Methode

Die 301 Patienten stimmten alle zu um innerhalb einer gleichen Periode 2 Mal am Tag das Haarwuchsmittel auf die Haupthaut anzubringen mittels einer kurzen leichten Massage. Das Medikament wurde hauptsächlich angewandt an Stellen wo die Rede war von Haarausfall wobei an Hand der Situation Stellen berücksichtigt wurden wo es kein Haarausfall gab.

Alle zwei Wochen wurden allen Patienten eine Flasche "Haarwuchsmittel" (100 ml) ausgereicht, wonach von einem medizinischen Spezialisten eine Untersuchung nach der klinisch wahrnehmbaren therapeutischen Effekten durchgeführt wurde; zugleich wurde vor der Behandlung, sowie je zwei Wochen während der Behandlungsdauer, Farbfotografien des Voransichtes, der beiden Seiten, des Hinteransichtes und des Scheitels gemacht.

Von 301 Fällen wurde vor Anfang der Behandung und nachdem die Behandlung 4 Wochen gedauert hatte, die Blutdurchstromkapazität von den kapillären Haargefässen von der Haupthaut gemessen. Hierbei wurde der "laser-Doppler"-Blutdurchstrommesser Type PF2 sowie einen "plotter" SE 120 angewandt um die Blutdurchstromkapazität der Haupthaut kwantitativ aufzunehmen, wobei die Einheiten der Blutdurchstromkapazität innerhalb dieser 9 Zonen festgestellt wurde und wurde für jede Zone einen "screening" von 30 Sekunden genommen. Danach wurde die also erhaltene Graphik der BLutdurchstromkapazität nach Analyse von einem Arzt mittels einen Computer weiter statistisch bearbeitet. Während der Messung wurde die Zimmertemperatur auf 20-24° C gehalten.

## Erfolge.

Nachdem die 301 Fälle das "Haarwuchsmittel" angewandt hatten, war nach einer Woche bei einer Minderheit die Rede von anfangendem Haarwuchs; nach zwei Wochen war bei 120 Fällen die Rede von Haarwuchs - 39,9% -, während nach 4 Wochen die Anzahl der Fälle wobei das Haar wieder gewachsen war auf 211 - 70,1% stieg.

Vor der Behandlung war die Blutdurchstromkapazität der kapillären Gefässe der Haupthaut auf die 9 Zonen durchschnittlich 335 ± 176 Millivolt (Tabelle J); von den 9 Zonen war im allgemeinen die durchschnittlichen Blutdurchstromkapazität von Punkt 1 und Punkt 6 deutlich niedriger als von Punkt 7 wo Haarausfall nicht leicht auftritt. $P<0.001 \sim 0.01$; für die durchschnittliche Blutdurchstromkapazität der übrigen Stellen war hier nicht die Rede von einer deutlichen Diskrepanz, $P \leq 0.05$.

Bei den verschiedenen Altersgruppen gab es keine statistischen Unterschiede der durchschnittlichen Blutdurchstromkapazität der 9 Punkte (Tabelle K); von den 258 männlichen Fällen war die durchschnittliche Blutdurchstromkapazität auf die 9 Punkte 335 ± 168 Millivolt, und von den 43 weiblichen Fällen war diese 333 ± 178 Millivolt, es gab hinsichtlich der Blutdurchstromkapazität der Haupthaut kein statistischer Unterschied zwischen männlichen und weiblichen Fällen. Nachdem das Haarwuchsmittel während 4 Wochen angewandt worden war wurde eine Vergleichung gemacht zwischen der Zustand der 9 Punkte auf die Haupthaut der 301 Fälle wie diese vor der Behandlung waren und nach der Behandlung während 4 Wochen. Es geht klar hervor dass in allen Fällen die Blutdurchstromkapazität der kapillären Gefässe der Haupthaut mit mehr als 100% zugenommen hatte - auf Basis einer statistischen Behandlung, P≤0.001; der Unterschied war extrem deutlich (Tabelle L). Nachdem der durchschnittlichen Blutdurchstromkapazität von 5 der 9 Punkte der unterschiedlichen Zonen, namentlich die Punkte 3, 4, 5, 7 und 8, verglichen war mit der von Punkt 1 ging hervor dass die Blutdurchstromkapazität der genannten 5 Punkte deutlich höher war als die vom Punkt 1.

Folgerung

1. Der "laser'Doppler"-Blutstrommesser ist ein am Anfang der 80-iger Jahren neu entwickeltes Instrument zur Bestimmung der Blutdurchstromkapazität der kapillären Gefässe der Haupthaut. Tenland und Nilson bestätigen mit einem hydraulischen Model, dass die ausgehenden Signale des obengenannten Apparates recht proportionel sind mit der Stromgeschwindigkeit der Erythrozyten und der Anzahl der Erythrozyten die durchstromt und dass es einen relativ guten linearen Karakteristik aufweist.

Der Bereich des Messungsgebietes des Instruments ist die Blutdurchstromkapazität der kapillären Gefässe von 1 mm dicken Haut. Das Instrument verursacht beim Messen keine Hautbeschädigung und es gibt keine Beschränkung hinsichtlich der Stelle der Messung; es verusacht kein Wärmeeffekt für die Haut; Der Zeitfaktor is gering, die Reaktionsfrequenz hoch und der Empfindlichkeitsgrad gut; die Messungen können ohne Probleme mehrmals durchgeführt werden; der Person der die Messung untergeht hat keine Schmerze; die Änderungen in der Dynamik können kontinuierlich observiert werden. Dies alles macht dass es jetzt international das meist ideale Instrument ist zur Untersuchung der Blutdurchstromkapazität. Bis jetzt war jedoch weder in China noch im Ausland ein Bericht erschienen betreffs die Anwendung dieses Instruments beim Untersuchen der Blutdurchstromkapazität von kapillären Gefässe der Haupthaut von mehreren hunderten Leidenden an Haarausfall.

2. Die Pathogenese von Haarausfall ist bis heute noch nicht klar. Nebst der neuropsychischen Theorie, der endocrine Theorie und der genetischen Theorie sind jetzt Wissenschaftler die die immunologische Theorie anführen, jedoch einen slechten Kreislauf und Blutzufuhr auf die Haupthaut gehören ungezweifeld auch zu den Faktoren die Haarausfall verursachen.

Bei der Analyse einer normalen Haupthaut ist gezeigt worden, dass die Haupthaut, wie die Haupt an anderen Stellen des Körpers, aus drei Schichten zusammengesetzt ist, namentlich die Epidermis, die Lederhaut und Unterhautbindegewebe. Zwischen der Epidermis und der Lederhaut befindet sich das höher gelegene Haargefässnetz und zwischen der Unterseite der Lederhaut und Unterhautsbindegewebe befindet sich das tiefer gelegene Haargefässnetz. Die Blutgefässe des tiefer gelegenen Haargefässnetzes gehen in vertikaler Richtung hoch, während die Verzweigungen das höher gelegene Haargefässnetz bilden.

Obgleich der Messung mit der "laser-Doppler" der Blutdurchstromkapazität von 1mm dicker Haut an erster Stelle die Blutdurchstromkapazität des höher gelegenen Haargefässnetzes wiedergeben wird, gibt die also erhaltene Information bezüglich der Zunahme oder Abnahme der Blutdurchstromkapazität selbstverständlich auch deutliche Information bezüglich der Zunahme oder Abnahme der Blutdurchstromkapazität des tiefer gelegenen Haargefässnetzes. Die Würzel der Haupthaare breiten sich aus zum Haarknopf mit einer konkaven Unterseite, wo das Bindegewebe der Lederhaut und das Unterhautbindegewebe tief eindringen und so die Haarpapille bilden. Darin befinden sich die Nervenende und auch die Blutgefässe die den Haarknopf ernähren, wodurch die für den normalen Haarwuchs notwendige Blutzufuhr aufrechterhalten wird.

3. Nach Anwendung des Haarwuchsmittels auf die 301 Persone, die an Haarverlust leiden, waren die Perioden innerhalb den das neue Haar zu wachsen anfing, durch die individuellen Unterschiede nicht gleich und nach 4 Wochen Awendung des Medikamentes gab es 211 Fälle, 70,1% der Versuchsperson, bei denen neues Haar gewachsen war. In der gleichen Zeit wurde die Blutdurchstromkapazität der kapillären Gefässe der Haupthaut von Versuchspersonen gemessen. Der Erfolg ergab dass bei einer Gesamtzahl von 301 Versuchspersonen an allen relevanten Stellen die Blutdurchstromkapazität der kapillären Gefässe der Haupthaut im Vergleich mit den gleichen Blutdurchstromkapazität vor der Behandlung, deutlich zugenommen war, mit einem Ratio von 100 %.

Nach 6 Wochen Behandlung war bei 60 Versuchspersonen neues Haar gewachsen, wodurch ins Gesamt bei 271 Versuchspersonen - 90,0% -, die Rede war von neuen Haarwuchs.

Bei diesen 60 Patienten war zuerst die Blutdurchstromkapazität der Haupthaut zugenommen und fing das Haar dann wieder an zu wachsen. Dies zeigt deutlich dass nach topikaler Anwendung des Haarwuchsmittels die normale Blutdurchstromkapazität der kapillären Gefässe der Haupthaut deutlich zunimmt, wonach die Nahrung und Blutzufuhr durch die Papillen der Haupthaut am Haarknopf verbessert, hierdurch werden die normalen physiologischen Funktionen wodurch Keratinbildung stattfindet, die bei den Patienten vor der Behandlung nicht mehr funktionerte mit als Erfolg Haarausfall, beschleunigt, welches neuen Haarwuchs verursachte.

4. Diese Untersuchung hat deutlich gezeigt, dass eine regelmässige Blutzufuhr nach der Mikrozirkulation auf der Haupthaut die physiologische Funktion ist die die Haarknöpfe aufrechterhält und dass ein regelmässiger Blutzufuhr einen wichtigen Faktor ist bei der Förderung von normalem Haarwuchs sowie bei der Wuchs von neues Haar nach Haarausfall.

Die Änderungen in der Blutdurchströmung auf alle betreffende Punkte der Haupthaut vor oder nach der Behandlung mit dem Haarwuchsmittel gemessen mit dem "laser-Doppler" Blutdurchstrommesser (durchschnittliche Werte ± Standarddeviation).

Tabelle J.

| Mess-Stelle | Blutstrom vor der Behandlung (mV) | Blutstrom 1 Monat nach der Behandlung (mV) | P-Wert |
|---|---|---|---|
| 1 | 325 ± 157 | 668 ± 269 | < 0.001 |
| 2 | 315 ± 165 | 702 ± 234 | < 0.001 |
| 3 | 381 ± 152 | 728 ± 252 | < 0.001 |
| 4 | 330 ± 182 | 781 ± 370 | < 0.001 |
| 5 | 317 ± 195 | 783 ± 236 | < 0.001 |
| 6 | 339 ± 165 | 697 ± 219 | < 0.001 |
| 7 | 385 ± 235 | 726 ± 212 | < 0.001 |
| 8 | 332 ± 145 | 719 ± 228 | < 0.001 |
| 9 | 358 ± 188 | 721 ± 240 | < 0.001 |
| durchschnittlich | 335 ± 23 | 725 ± 37 | < 0.001 |

**Tabelle K. Die durchschnittliche Blutdurchströmung von den 9 Punkten der Haupthaut eingeteilt nach Alter. (Durchschnittlicher Wert ± Standard Deviation in Millivolt).**

```
Mess-
Stelle    ≤20 J              21-30 J            31-40 J     41-50 J    51-60 J
1         254 ± 59           319 ± 19           331 ± 16    327 ± 14   280 ± 39
2         204 ± 31           309 ± 19           316 ± 13    326 ± 19   283 ± 42
3         245 ± 46           338 ± 20           306 ± 12    321 ± 17   265 ± 50
4         348 ± 103          321 ± 21           301 ± 12    365 ± 21   382 ± 131
5         380 ± 38           294 ± 18           304 ± 18    345 ± 21   276 ± 69
6         388 ± 86           319 ± 19           328 ± 12    360 ± 21   349 ± 37
7         349 ± 80           381 ± 19           357 ± 15    421 ± 32   393 ± 67
8         370 ± 35           328 ± 16           335 ± 13    330 ± 16   304 ± 42
9         415 ± 21           344 ± 20           360 ± 20    357 ± 16   403 ± 51
durch-
schnitt-
lich      328 ± 55           328 ± 19           327 ± 15    350 ± 20   307 ± 59
```

Tabelle L.

| Änderungen des Blutstromes in der Mikrozirkulation der Haupthaut nach 1 Monat Anwendung des Haarwuchsmittels bei 301 Versuchspersonen. | | |
|---|---|---|
| Mess-Stelle | vor der Behandlung | nach der Behandlung | p-Wert |
| 1 | 325 ± 157 | 668 ± 269 | <0.001 |
| 2 | 315 ± 165 | 702 ± 234 | <0.001 |
| 3 | 318 ± 152 | 728 ± 252* | <0.001 |
| 4 | 330 ± 182 | 781 ± 370** | <0.001 |
| 5 | 317 ± 195 | 783 ± 236** | <0.001 |
| 6 | 339 ± 165 | 697 ± 219 | <0.001 |
| 7 | 385 ± 235* | 726 ± 212* | <0.001 |
| 8 | 332 ± 145 | 719 ± 228 | <0.001 |
| 9 | 358 ± 188 | 721 ± 240* | <0.001 |
| durchschittlich | 335 ± 176 | 725 ± 251 | <0.001 |
| Die anderen Punkte im Verleich mit Punkt 1: | | | |

\* P 0.01
\*\* P 0.001

Das Gemische wurde von einer grossen Anzahl von Versuchspersonen angewandt durch zwei Mal am Tag einmassieren von etwa 3,5 ml. Die Erfolge wurden monatlich beurteilt. In der Tabelle M sind die Personen mit ihren Altern angegeben worden, welche Photographien vor der Behandlung genommen wurden und welche Photografien nach einem Monat, nach zwei Monaten usw. genommen wurden.

Tabelle M

| Name | Alter | Photografien |
|---|---|---|
| Frau A. | 37 | 1 und 2 |
| Fräulein B | 18 | 1,2 und 3 |
| Fräulein C | 21 | 1,2 und 3 |
| Frau D | 44 | 1,2,3 und 4 |
| Fräulein E | 23 | 1 und 2 |
| Frau F | 36 | 1,2 und 3 |
| Fräulein G | 34 | 1,2,3 und 4 |
| Fräulein H | 54 | 1,2,3 und 4 |
| Herr I | 44 | 1,3 und 4 |
| Herr J | 21 | 1,2,3,4 und 5 |
| Herr K | 31 | 1,2,3,4 und 5 |
| Herr L | 4 | 1,2,3, und 4 |
| Herr M | 36 | 1,2,3, und 4 |
| Herr N | 35 | 1,2,3, und 4 |
| Herr O | 36 | 1,2 und 3 |
| Herr P | 37 | 1,2,3,4 und 5 |

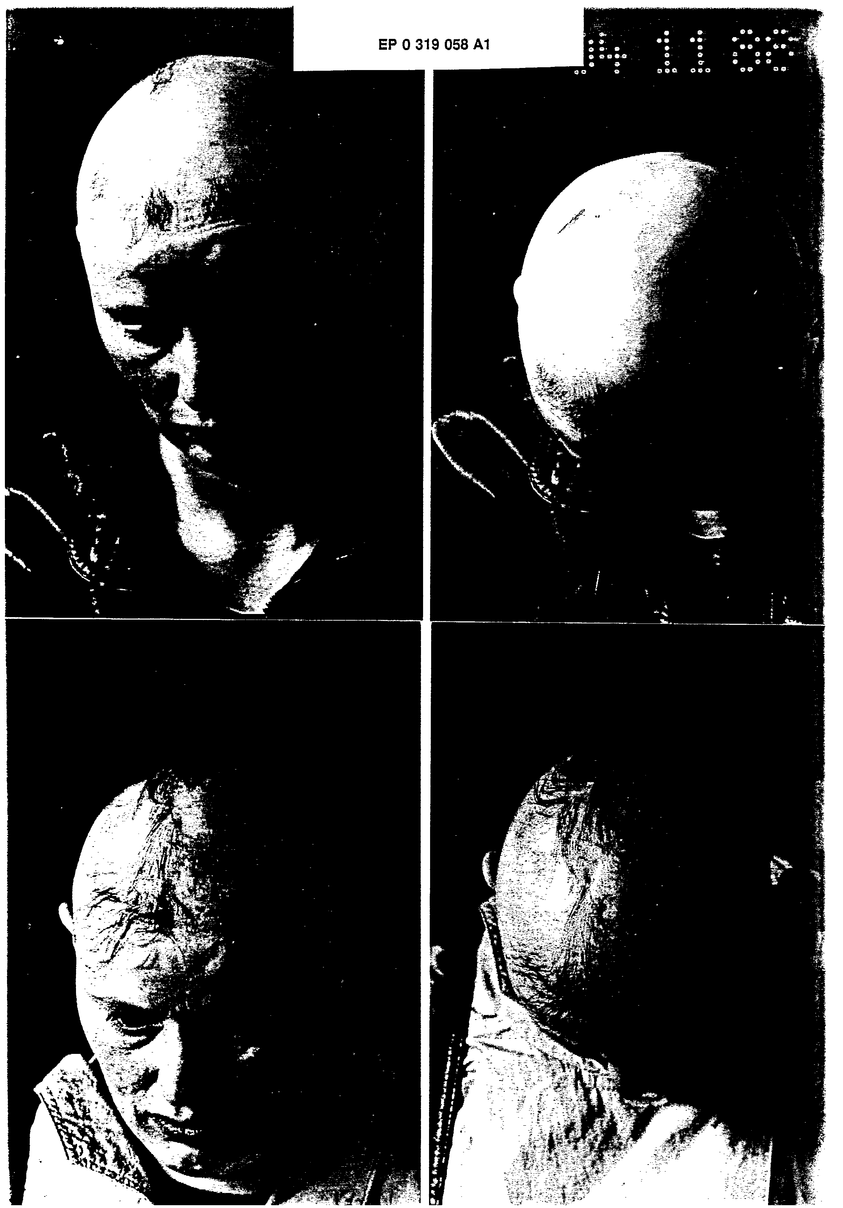

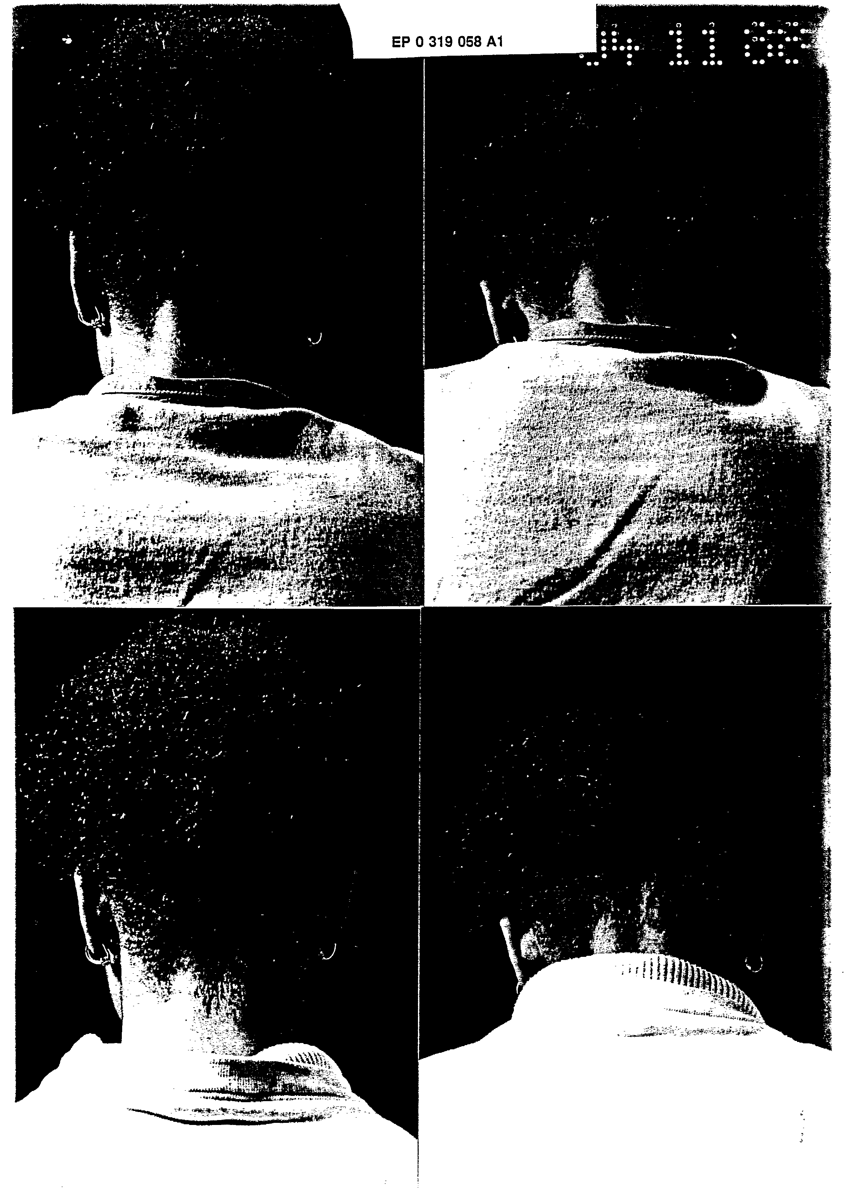

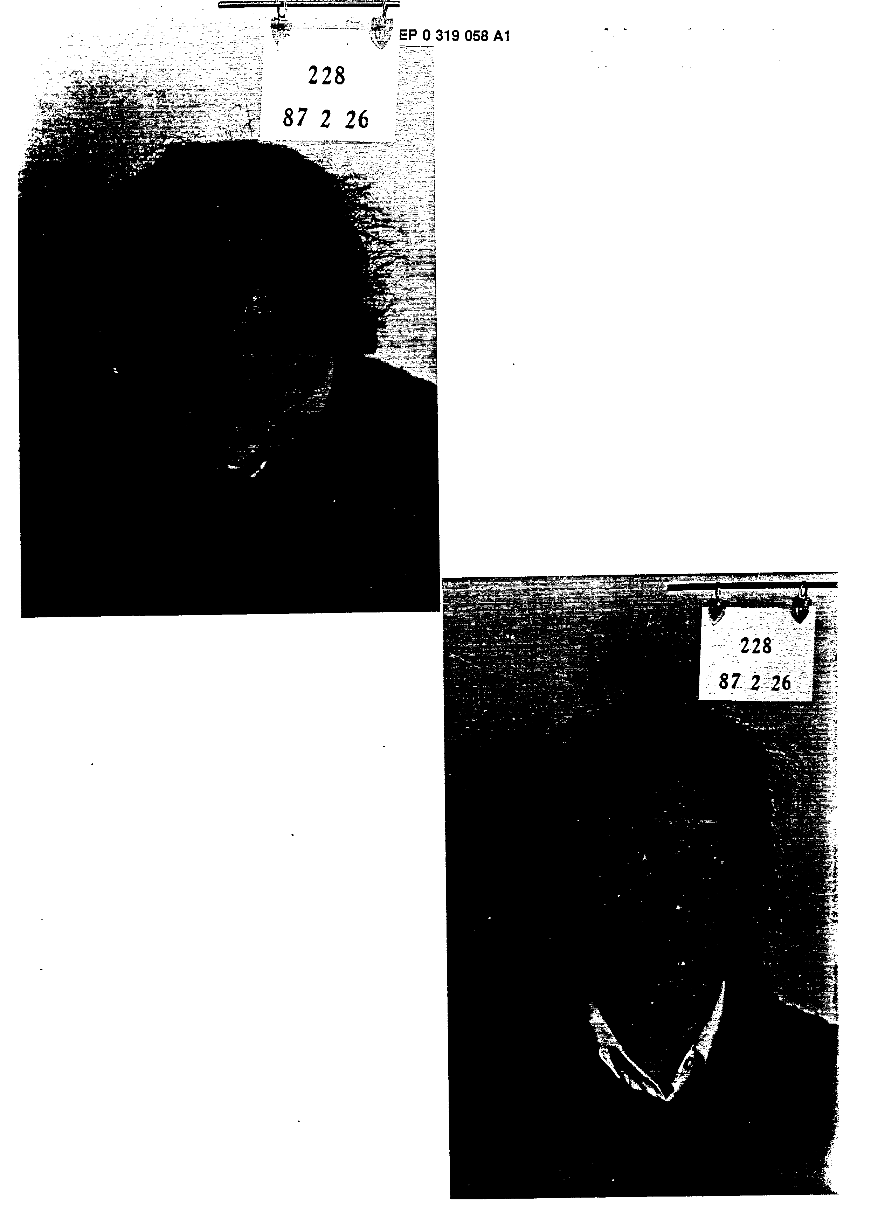

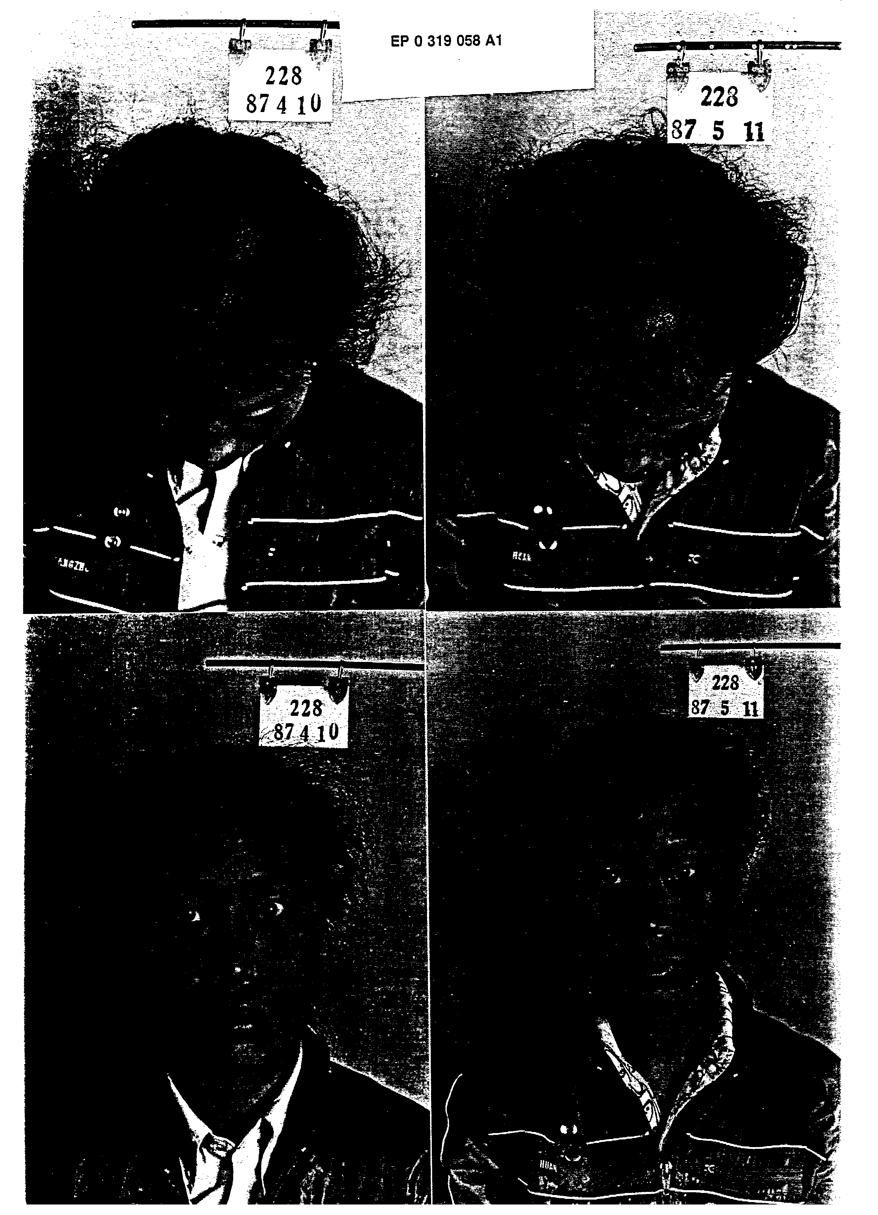

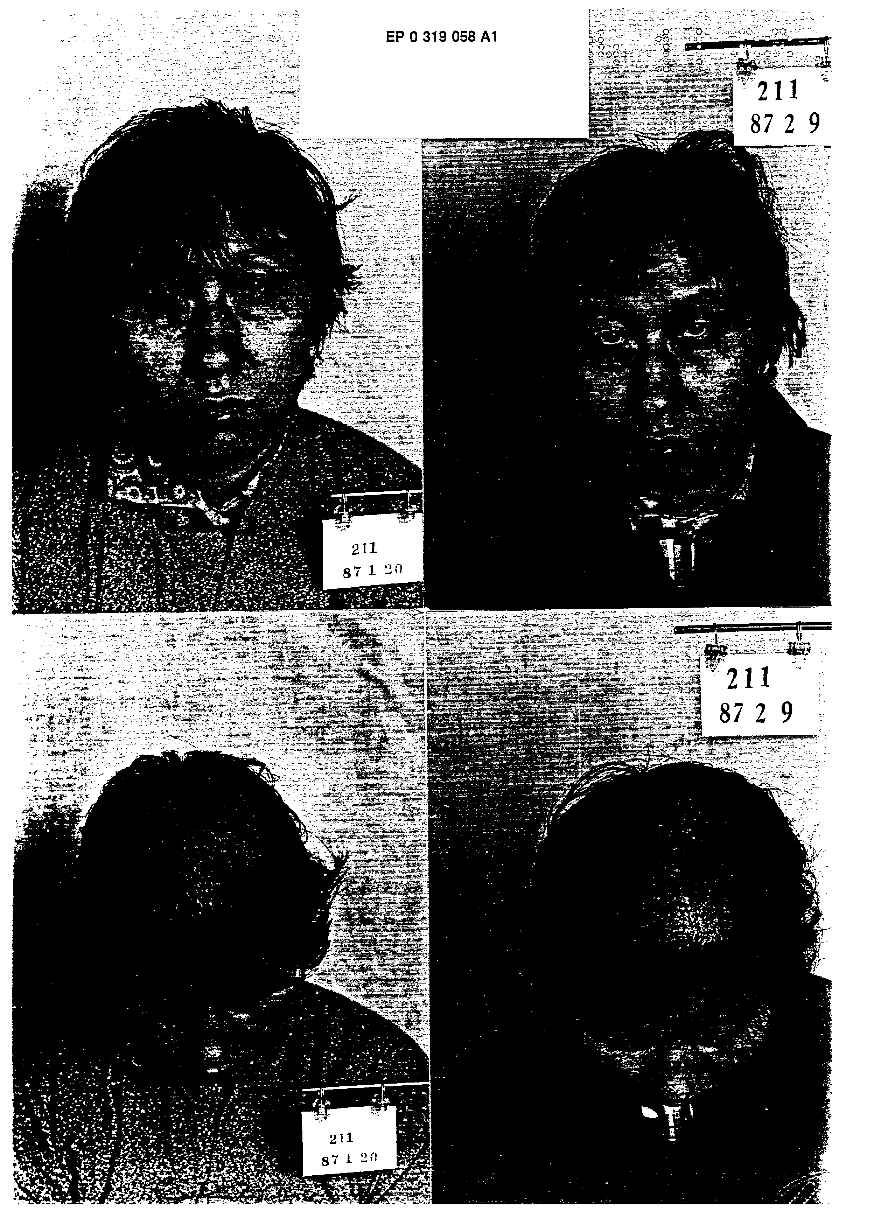

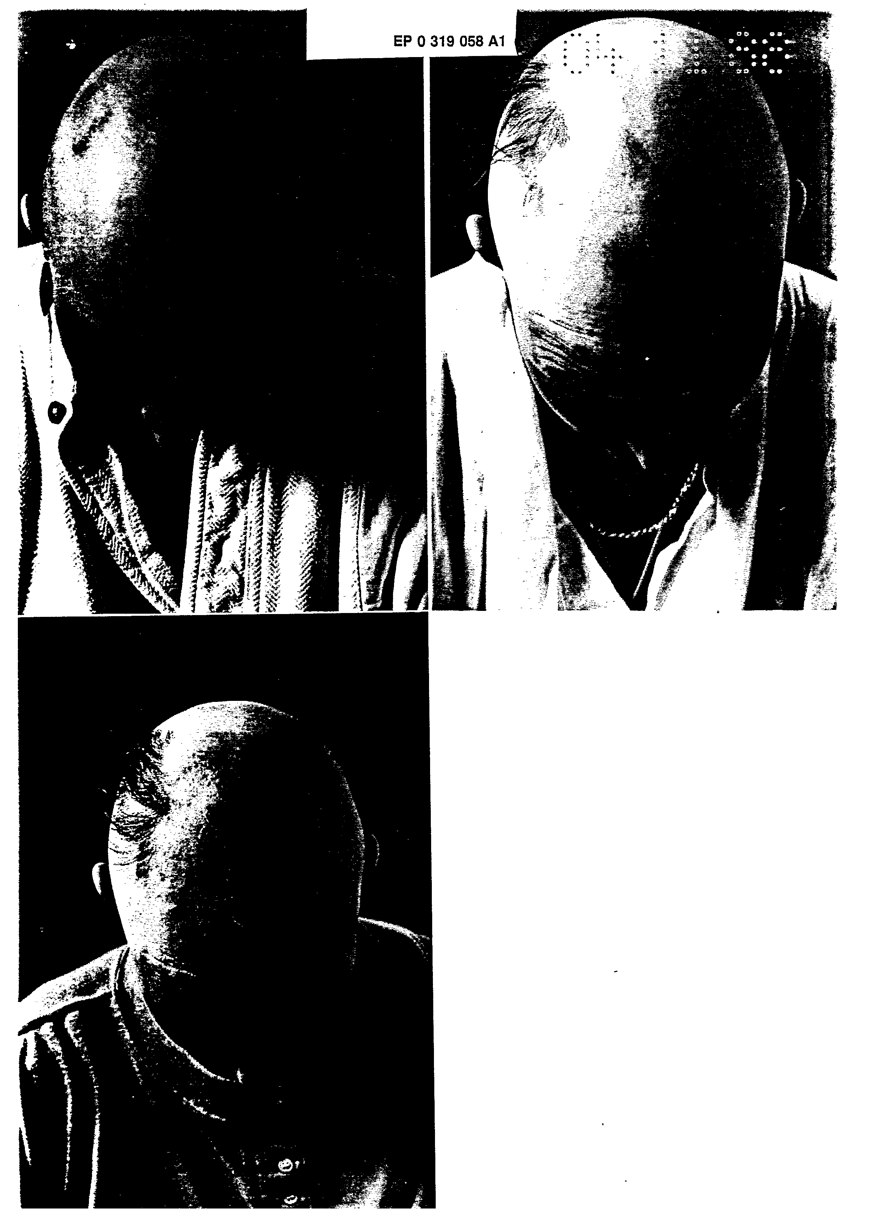

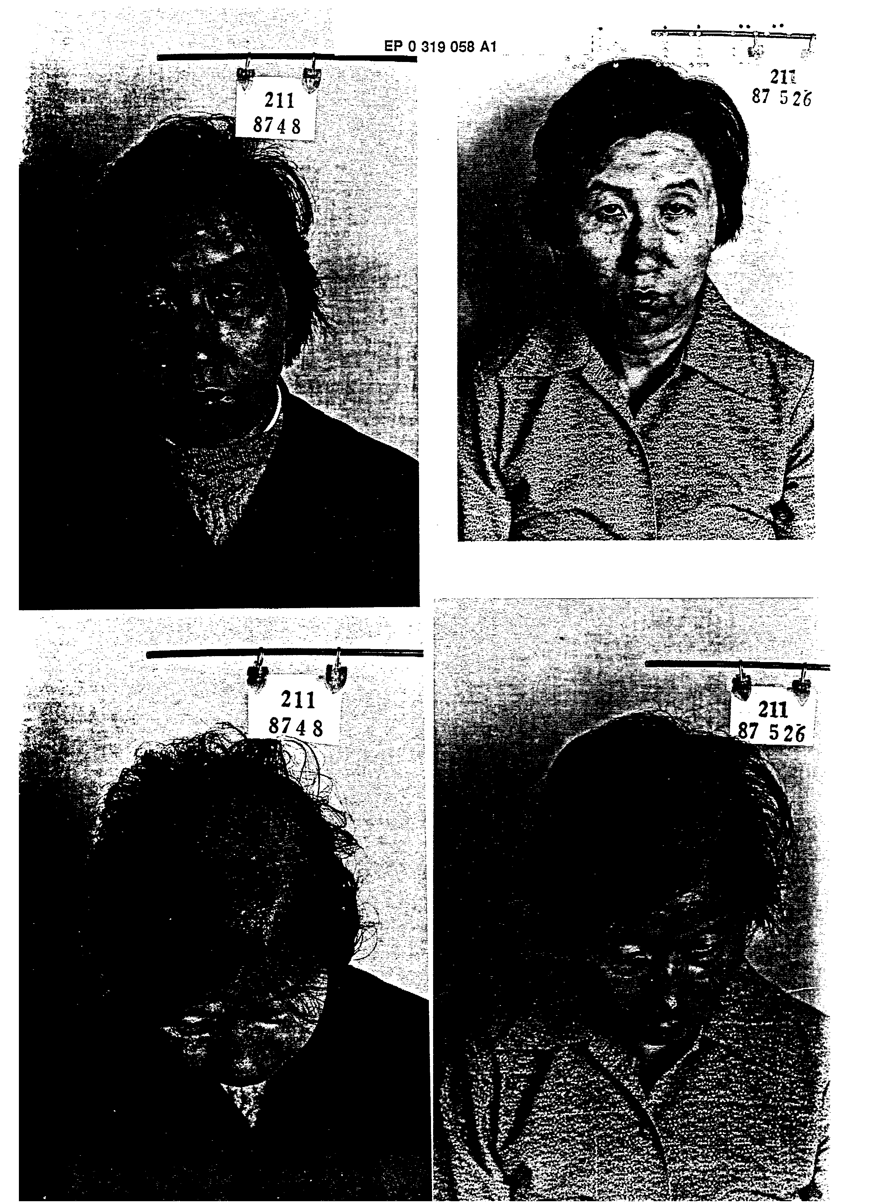

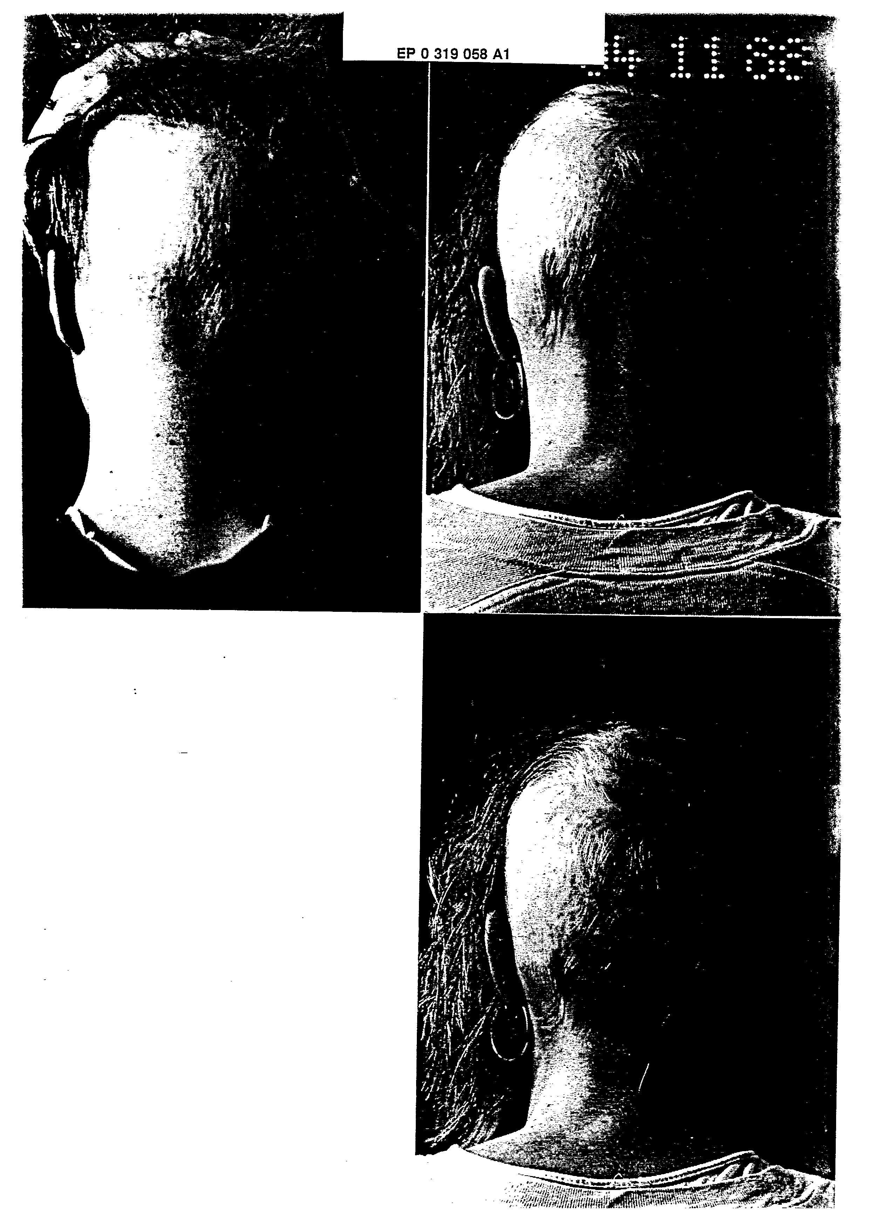

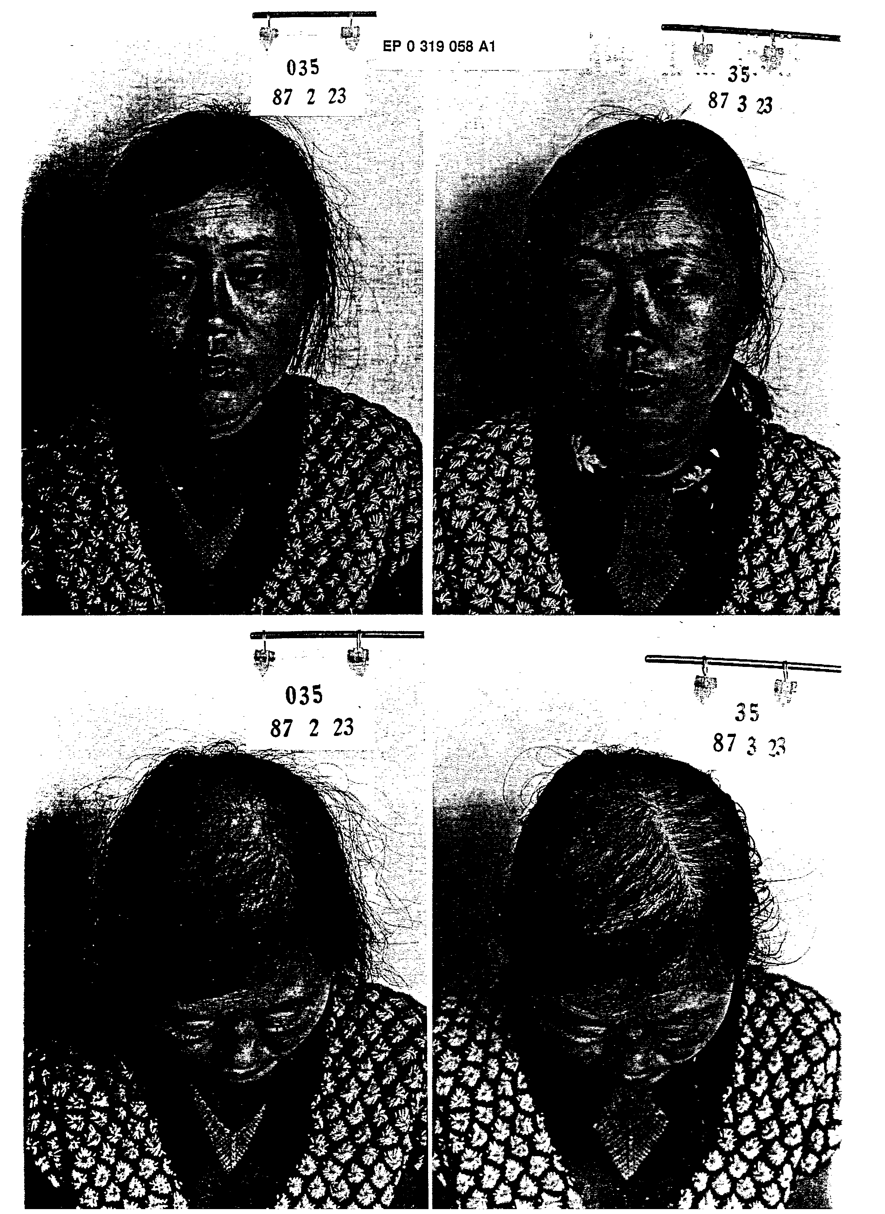

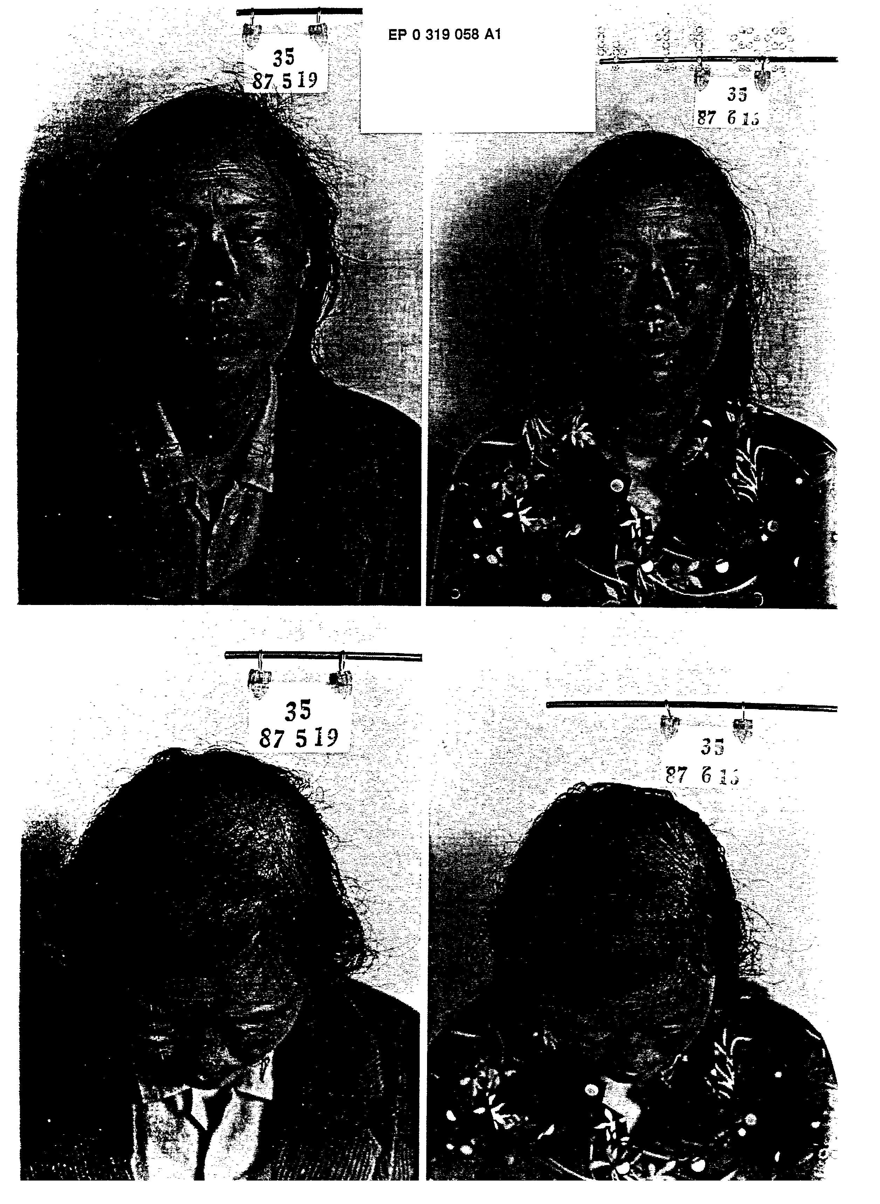

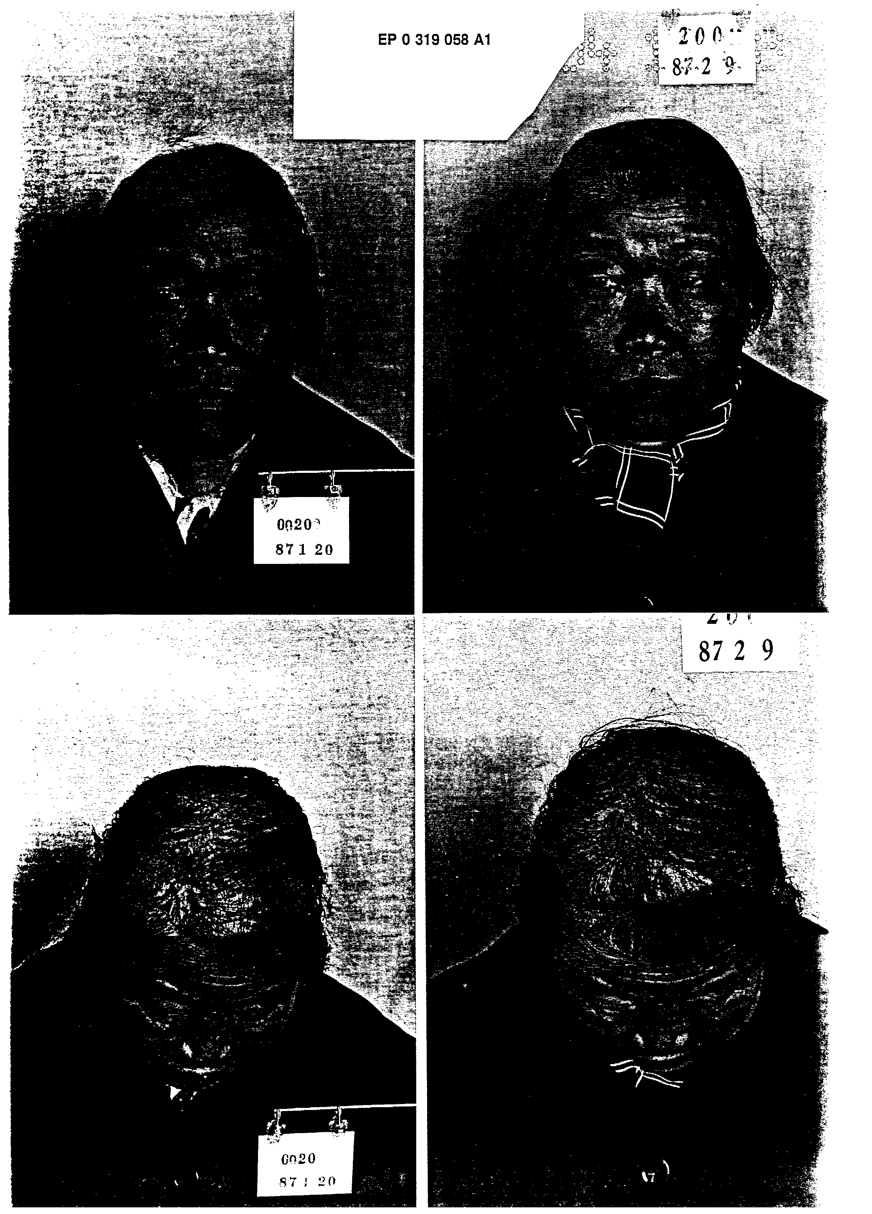

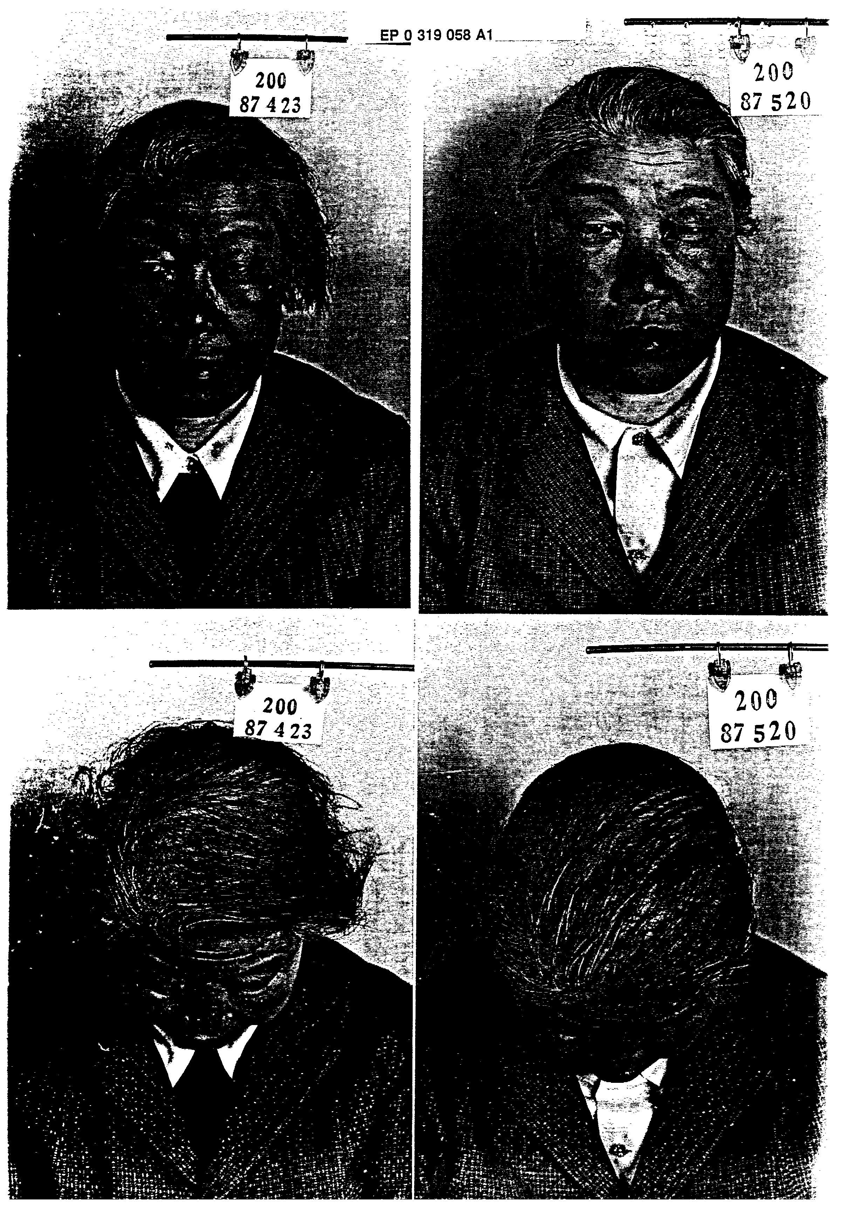

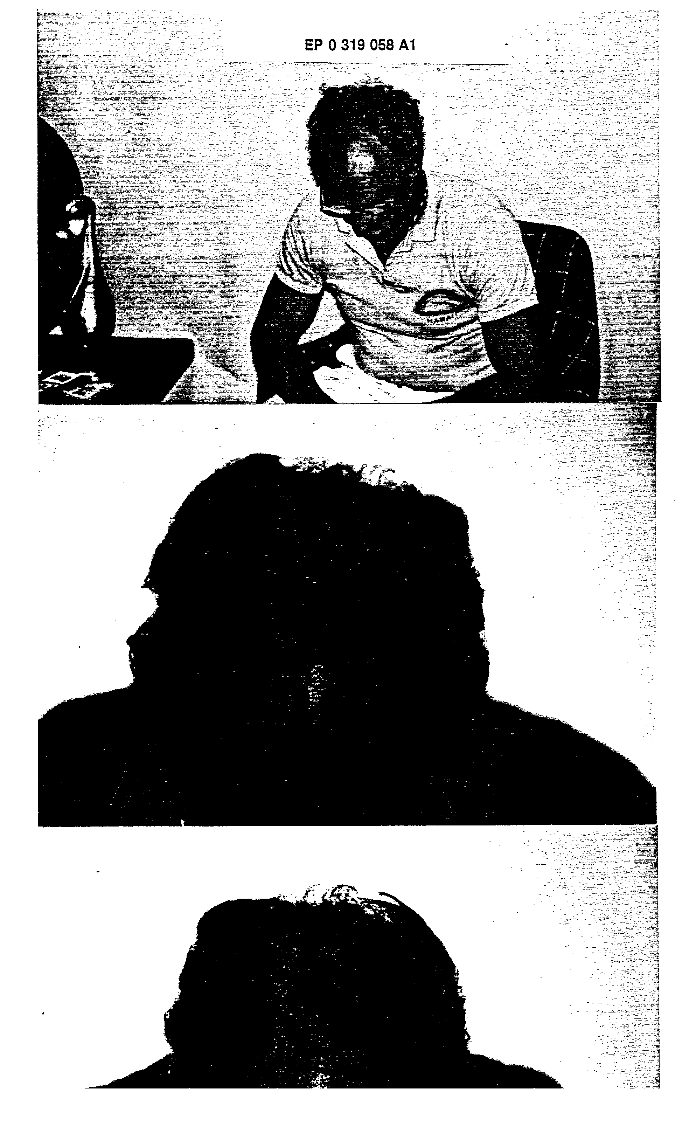

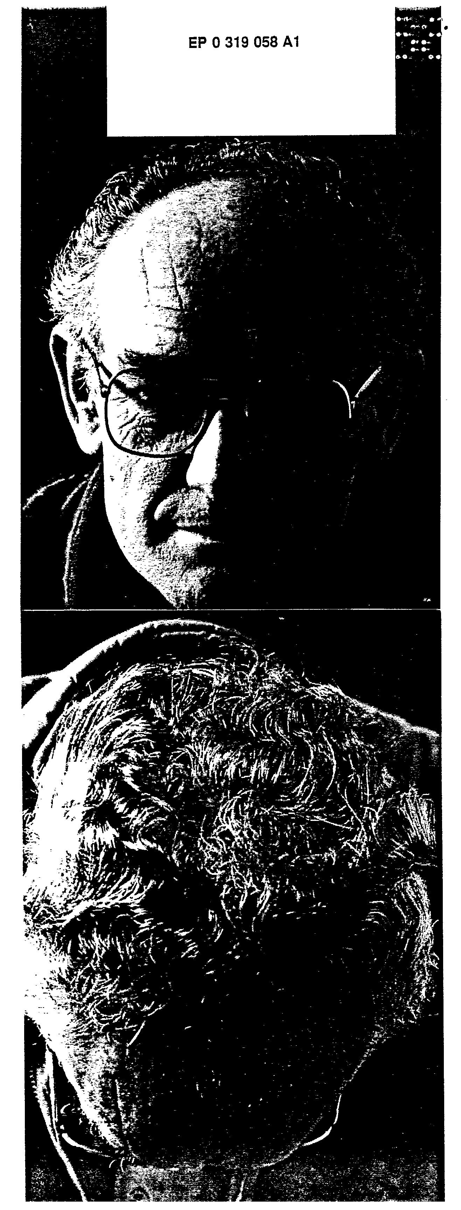

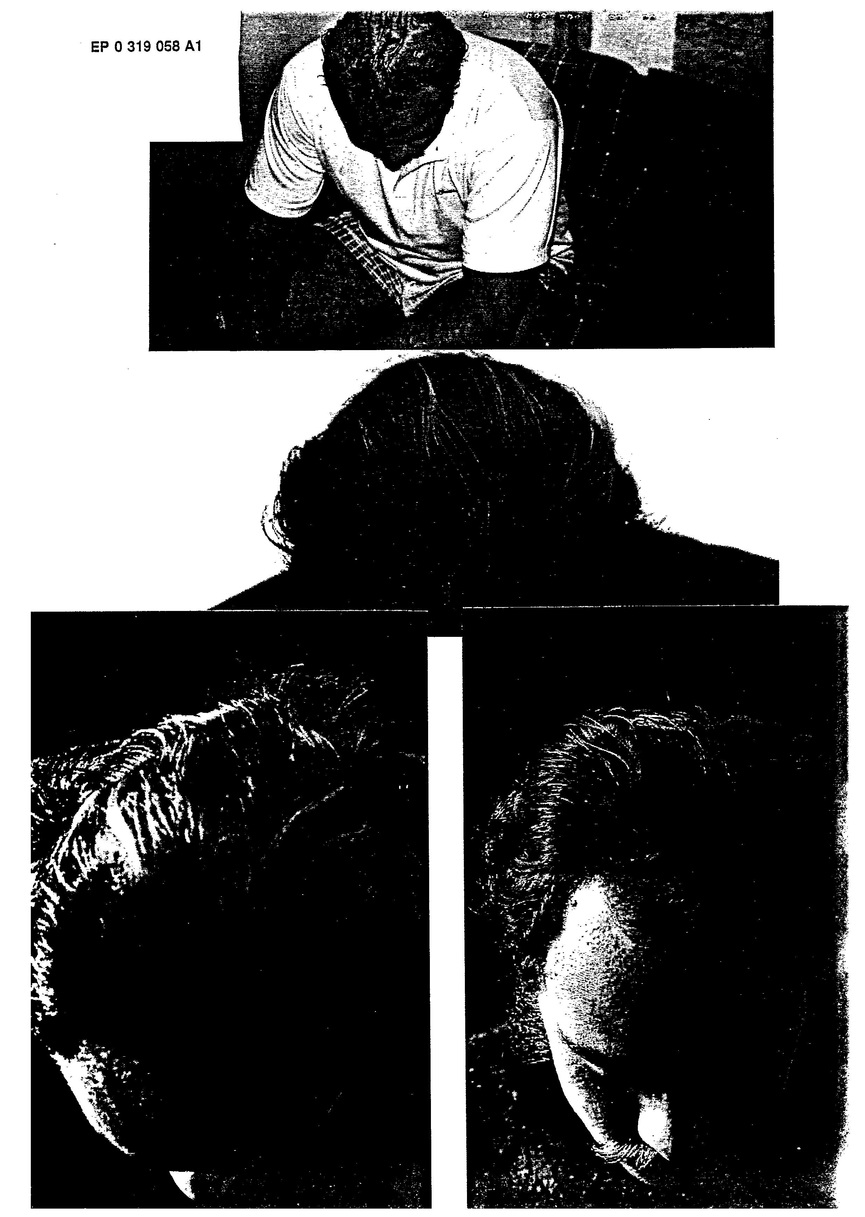

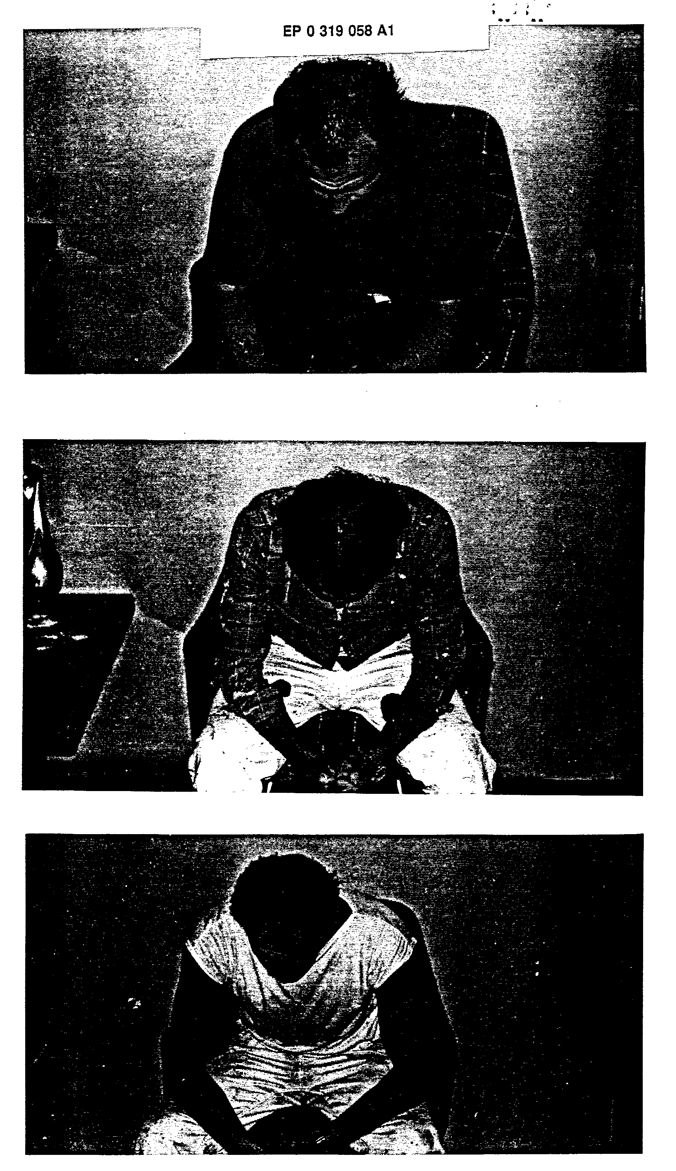

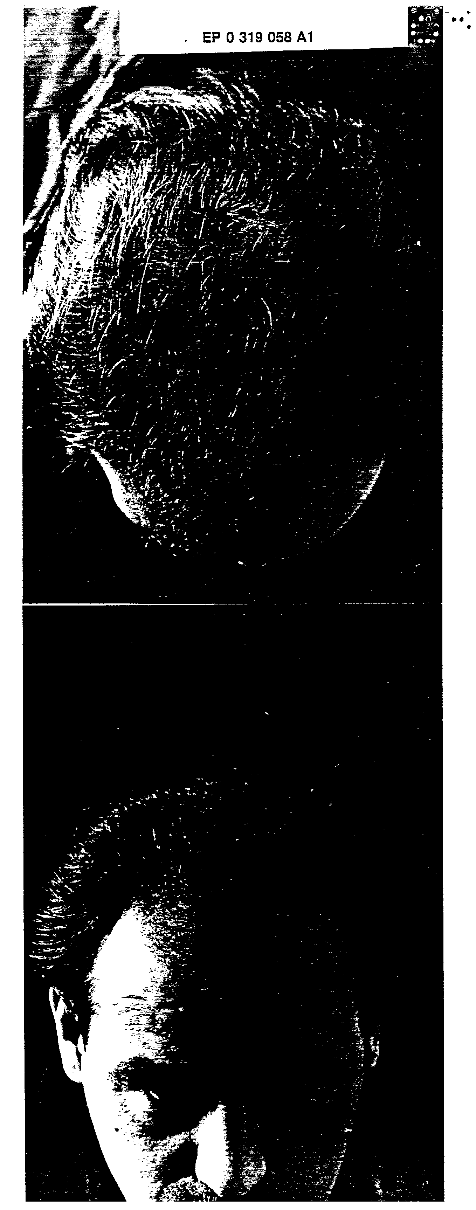

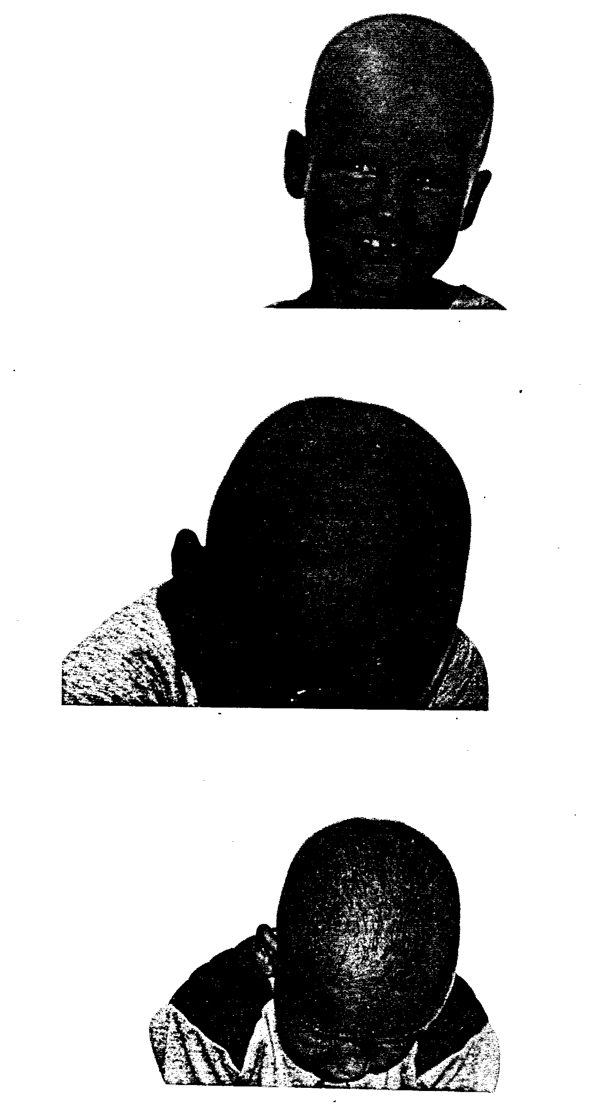

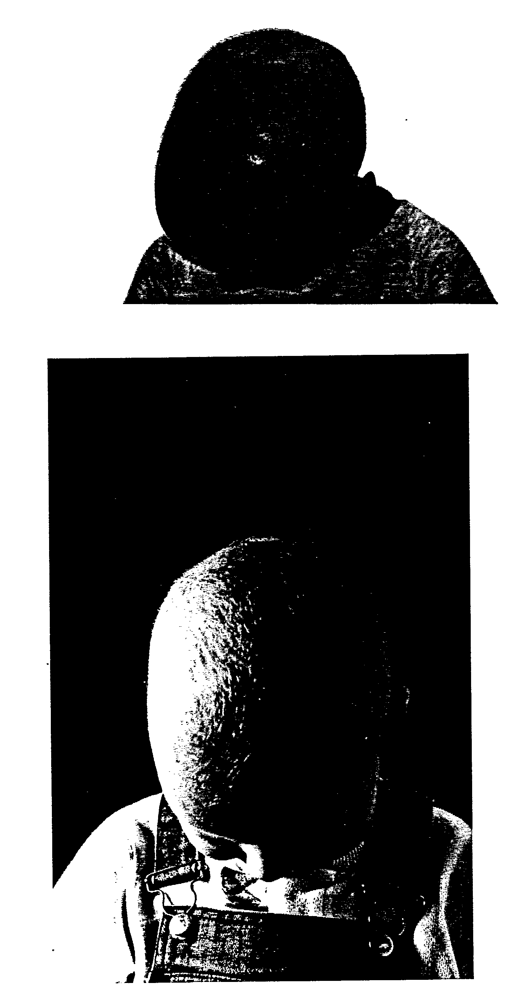

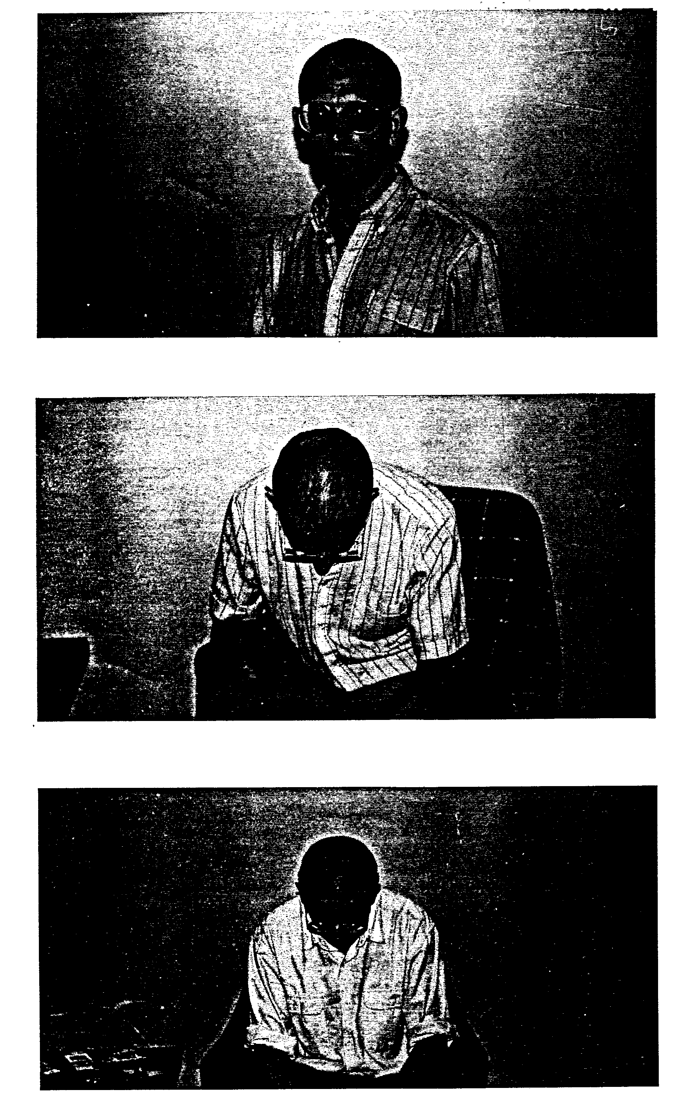

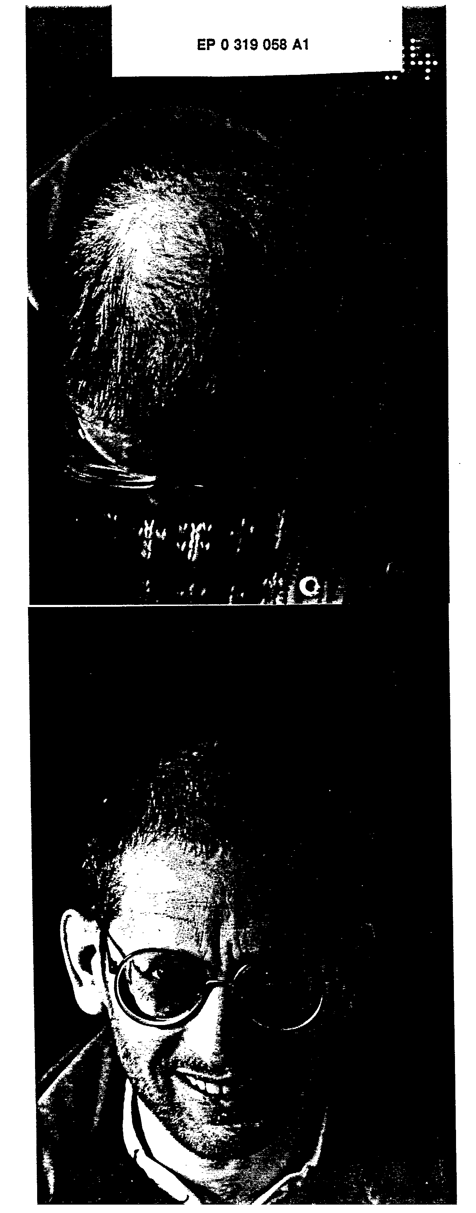

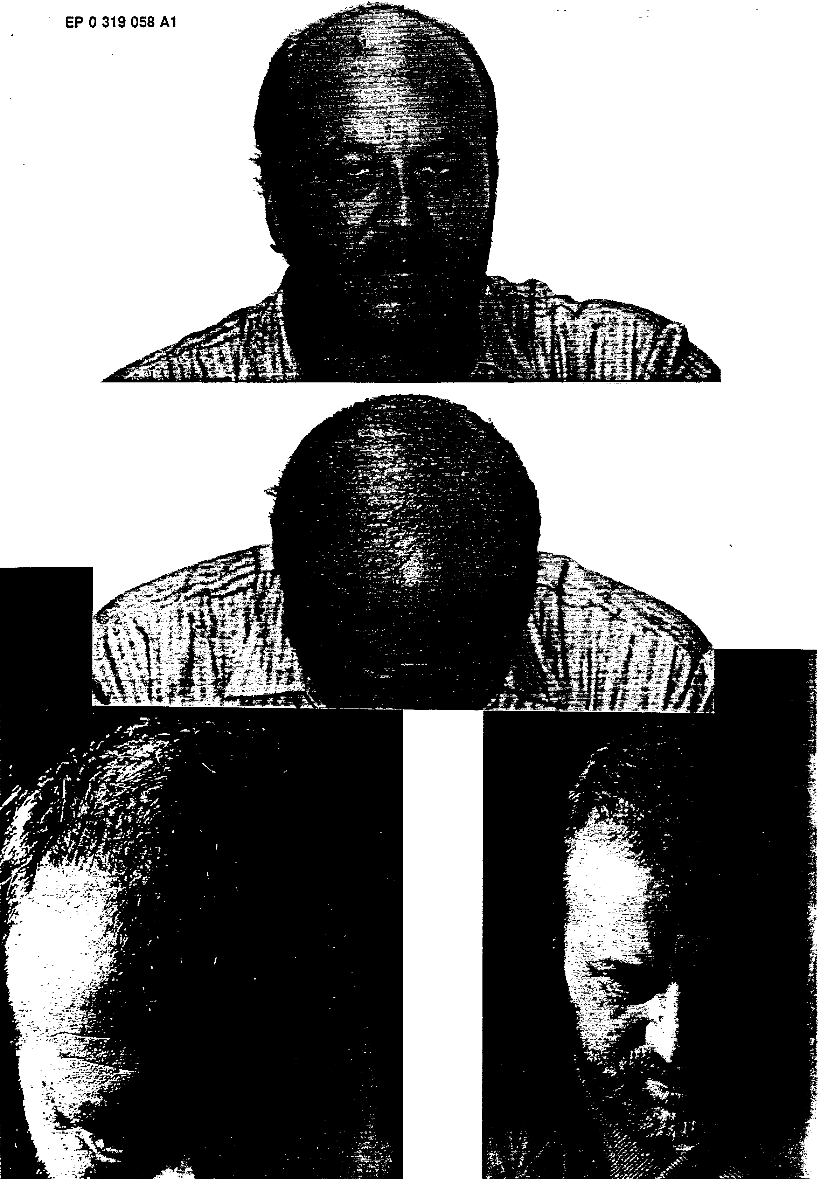

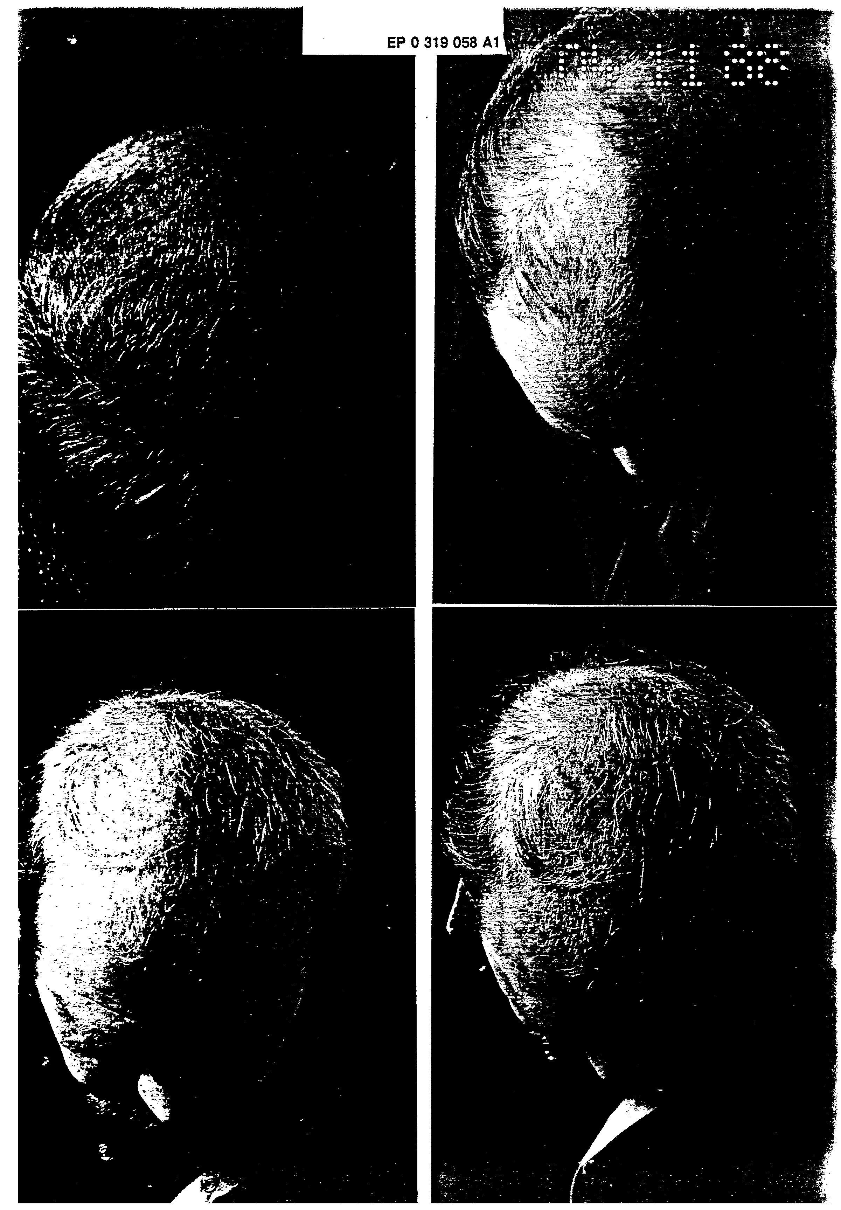

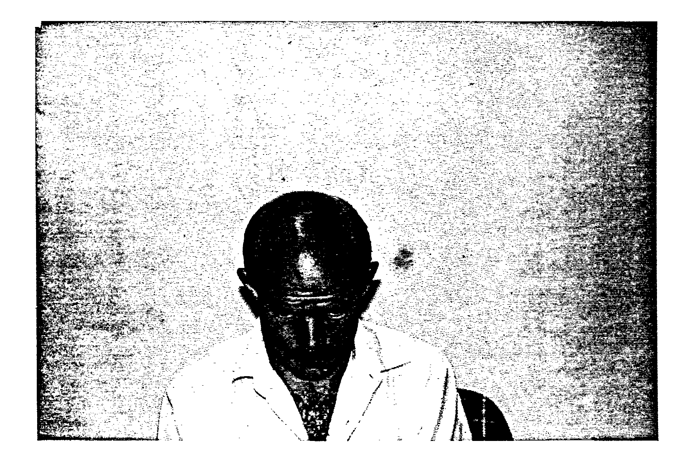

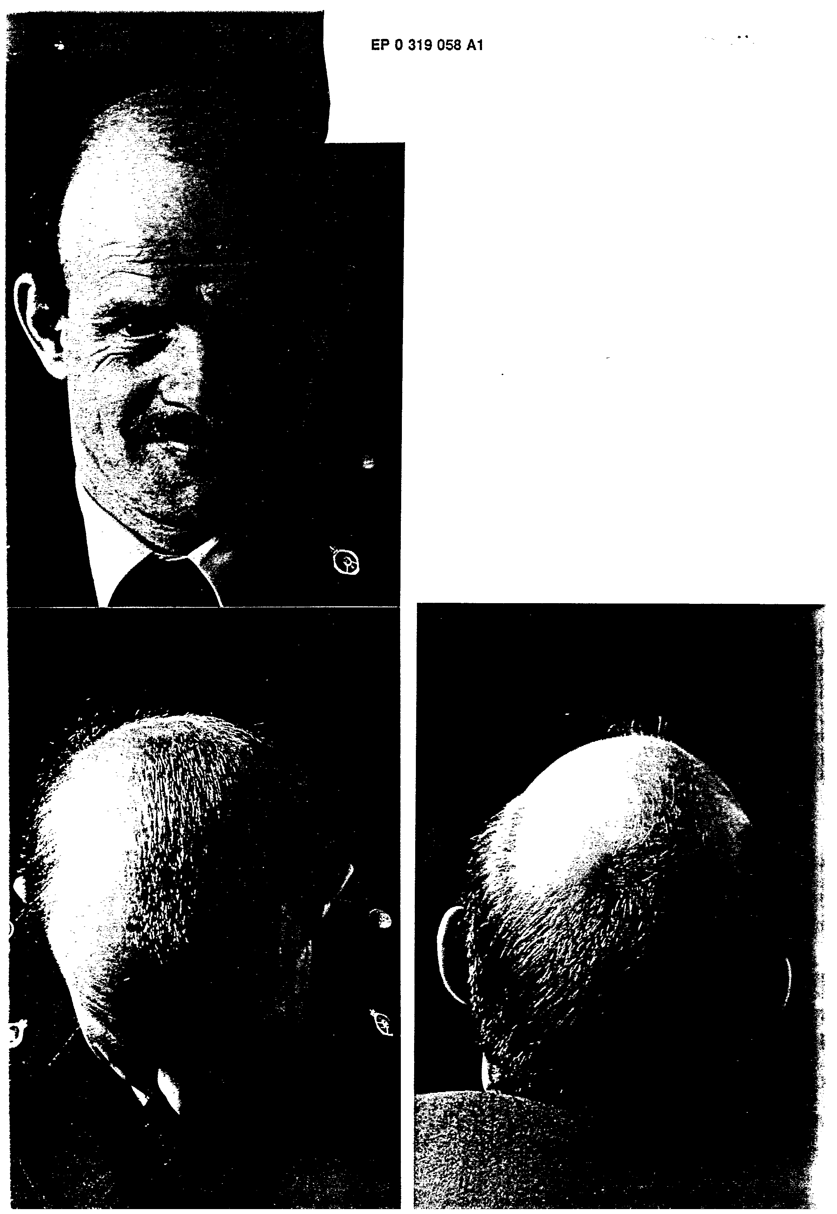

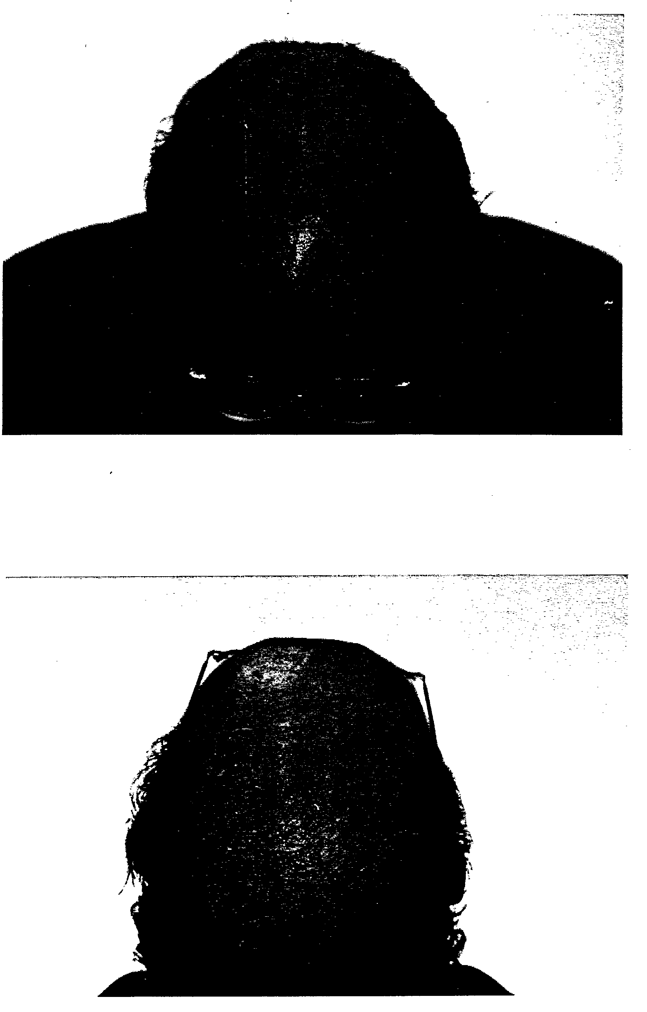

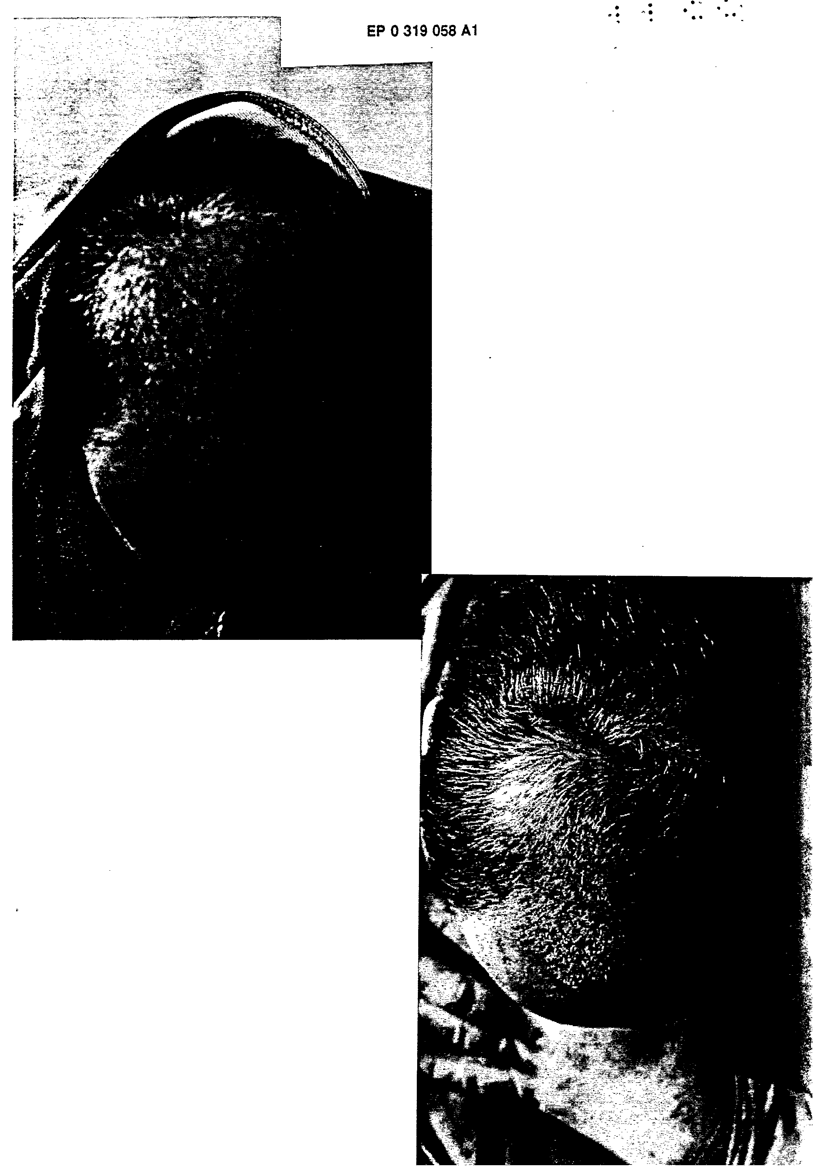

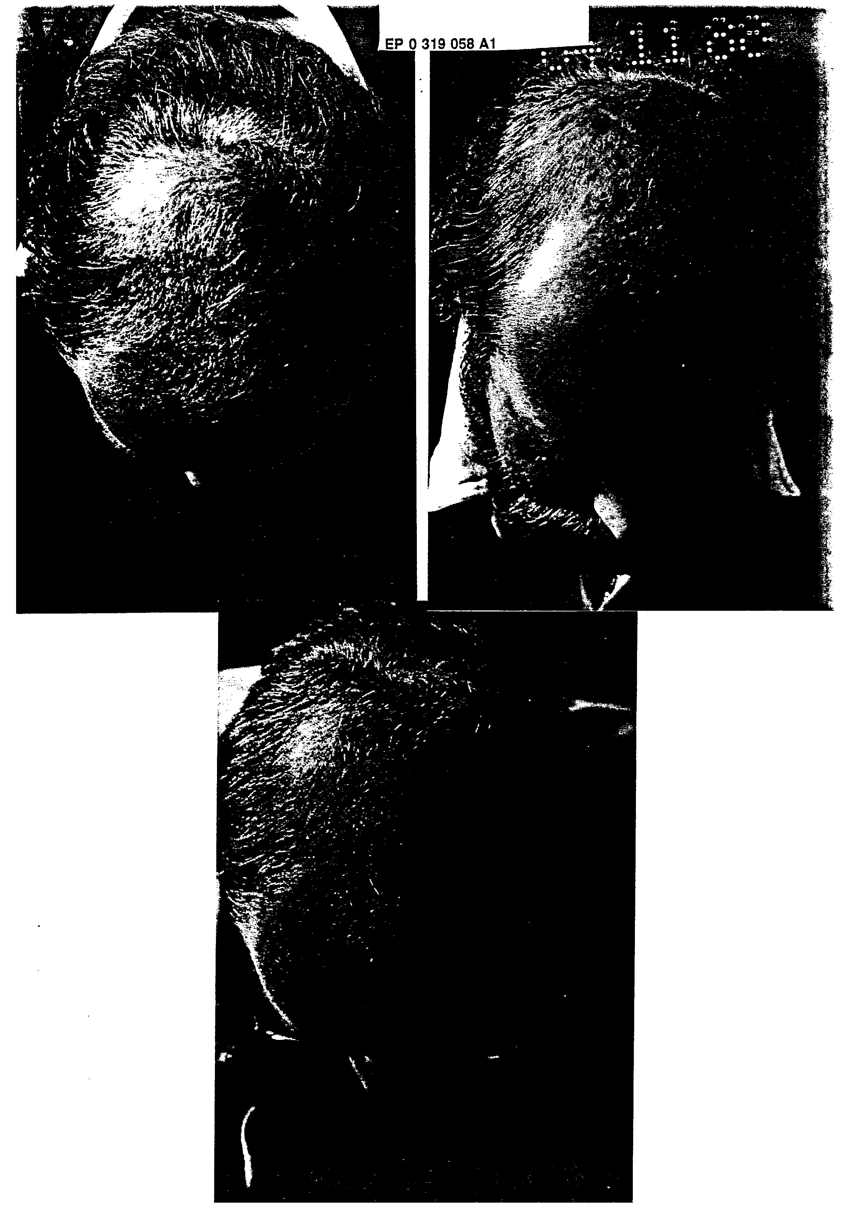

**Ansprüche**

1. Mittel zur Förderung des Haarwuchses, das pflanzenartige Extrakte aufweist, dadurch gekennzeichnet, dass dieses Mittel hergestellt worden ist auf Basis eines Extraktes erhältlich durch Extraktion von:

3 - 7 Gew. Tln Sichuan Pericarpium zanthoxyli;

7 - 11 Gew. Tln Radix Angelicae sinensis;

7 - 11 Gew. Tln Cacumen biotae;

4 - 8 Gew. Tln Folium Mori;

7 - 11 Gew. Tln Rhizoma Zingiberis recens;

0,5 - 1,5 Gew. Tln Radix Stemonae

15 - 25 Gew. Tln Fructus Grataegi;

2,5 - 3,5 Gew. Tln Flos.Carthami;

0,75 - 1,25 Gew.Tln Fructus Capsici;

4 - 8 Gew. Tln Folium Sesami;

2,5 - 3,5 Gew. Tln Cortex Pseudolaricis;

mit 100 Gew. Tln eines Gemisches aus 50-70 Gew. % Äthanol und 30-50 Gew.% Wasser und worin

1,5 - 2,5 mg Quecksilbersulfat;

7,5 - 12,5 mg Bleisulfat und

2,5 - 3,5 mg Arsensulfat pro 100 ml und gegebenenfalls weitere Verdünnungsmittel, Riechstoffe, Farbstoffe und/oder Verdickungsmittel aufgenommen worden sind.

2. Mittel gemäss Anspruch 1, dadurch gekennzeichnet dass dieses Mittel hergestellt ist auf Basis eines Extraktes erhältlich durch Extraktion von:

4,5 - 5,5 Gew. Tln Sichuan Pericarpium Zanthoxyli;

8,5 - 9,5 Gew. Tln Radix Angelicae Sinensis;

8,5 - 9,5 Gew. Tln Cacumen Biotae;

5,5 - 6,5 Gew. Tln Folium Mori;

8,5 - 9,5 Gew. Tln Rhizoma Zingiberis Recens;

0,9 - 1,1 Gew. Tln Radix Stemonae;

19 - 21 Gew. Tln Fructus Grataegi;

2,8 - 3,2 Gew. Tln Flos. Carthami;

0,9 - 1,1 Gew. Tln Fructus Capsici;

5,5 - 6,5 Gew. Tln Folium Sesami;

2,9 - 3,1 Gew. Tln Gortex Pseudolaricis;

mit 100 Volumenteilen eines Äthanol-Wasser-Gemisches zu extrahieren und worin:

1,9 - 2,1 mg Quecksilbersulfat;

9,5 - 10,5 mg Bleisulfat

2,9 - 3,1 mg Arsensulfat pro 100 ml und gegebenenfalls weitere Verdünnungsmittel, Riechstoffe, Farbstoffe und oder Verdickungsmittel aufgenommen worden sind.

3. Mittel gemäss Anspruch 2, dadurch gekennzeichnet dass dieses Mittel hergestellt ist auf Basis eines Extraktes erhältlich durch Extraktion von:

5 Gew. Tln Sichuan Pericarpium zanthoxyli;

9 Gew. Tln Radix Angelicae sinensis;

9 Gew. Tln Cacumen biotae;

6 Gern. Tln Folium mori;

9 Gew. Tln Rhizoma Zingiberis recens;

1 Gew. Tl Radix Stemonae;

20 Gew. Tln Fructus Grataegi;

3 Gern. Tln Flos. Carthami;

1 Gew. Tl Fructus Capsici;

6 Gew. Tln Folium Sesami;

3 Gew. Tln Gortex Pseudolaricis;

mit 100 Volumenteilen eines Äthanol-Wassergemisches worin:

0,02 mg Quecksilbersulfat;

0,1 mg Bleisulfat;

0,03 mg Arsensulfat pro 100 ml und gegebenenfalls weitere Verdünnungsmittel, Riechstoffe, Farbstoffe und/oder Verdickungsstoffe aufgenommen worden sind.

4. Mittel gemäss Ansprüche 1-3, dadurch gekennzeichnet dass dieses 60-70 Gew.% Alkohol und 30-40 Gew.% Wasser enthält.

EP 0 319 058 A1

EP 0 319 058 A1

211
87 2 9

211
87 1 20

211
87 2 9

211
87 1 20

EP 0 319 058 A1

035
87 2 23

35
87 3 23

035
87 2 23

35
87 3 23

EP 0 319 058 A1

EP 0 319 058 A1

EP 0 319 058 A1

EP 0 319 058 A1

EP 0 319 058 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB-A-2 095 553 (INTERAG RT) * Ansprüche; Beispiele * --- | 1 | A 61 K 7/06 |
| A | SEIFEN-ÖLE-FETTE-WACHSE, Band 107, Nr. 20, 10. Dezember 1981, Seiten 623-625, Augsburg, DE * Seiten 624-625, "Haarkosmetik" * ----- | 1 | |

RECHERCHIERTE SACHGEBIETE (Int. Cl.4)

A 61 K

Der vorliegende Recherchenbericht wurde für alle Patentansprüche erstellt

| Recherchenort | Abschlußdatum der Recherche | Prüfer |
|---|---|---|
| DEN HAAG | 13-03-1989 | WILLEKENS G.E.J. |

EPO FORM 1503 03.82 (P0403)